# EUROPÄISCHE PATENTSCHRIFT

(11) **EP 0 661 282 B1**
(45) Veröffentlichungstag und Bekanntmachung des Hinweises auf die Patenterteilung: **22.04.1998**
(21) Anmeldenummer: 94119743.6
(22) Anmeldetag: 14.12.1994
(51) Int. Cl.: C07D 471/04, C07D 491/04, C07D 213/80, A01N 43/40

(54) **Pyridin-2,3-dicarbonsäureimide, Verfahren zu ihrer Herstellung und ihre Verwendung zur Bekämpfung unerwünschten Pflanzenwuchses**
Pyridine-2,3-dicarboxylic imides, process for their preparation and their use to control the growth of unwanted plants
Pyridine-2,3-dicarboxylique imides, procédé pour leur préparation et leur utilisation pour réprimer la croissance de plantes indésirables

(30) Priorität: 22.12.1993 DE 4343923
(43) Veröffentlichungstag der Anmeldung: 05.07.1995
(73) Patentinhaber: BASF AKTIENGESELLSCHAFT, 67056 Ludwigshafen (DE)
(72) Erfinder: Hamprecht, Dr. Gerhard, D-69469 Weinheim (DE); Fuchs, Dr. Eberhard, D-67227 Frankenthal (DE); Gerber, Dr. Matthias, D-67117 Limburgerhof (DE); Walter, Dr. Helmut, D-67283 Obrigheim (DE); Westphalen, Dr. Karl-Otto, D-67346 Speyer (DE)

(56) Entgegenhaltungen:
- EP-A- 0 041 623
- EP-A- 0 128 006
- EP-A- 0 422 456
- GB-A- 2 174 395

## Beschreibung

Die vorliegende Erfindung betrifft Pyridin-2,3-dicarbonsäureimide der allgemeinen Formel I 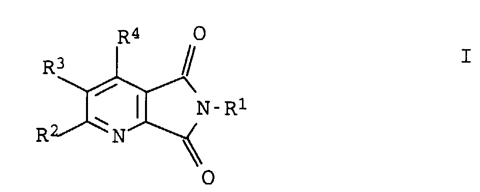 in der
R¹ Wasserstoff;
C₁-C₄-Alkoxy;
C₁-C₆-Alkyl, das eine bis drei der folgenden Gruppen tragen kann: C₁-C₄-Alkoxy, C₁-C₄-Halogenalkoxy, C₁-C₄-Alkylthio, C₁-C₄-Halogenalkylthio, C₁-C₄-Dialkylamino, C₃-C₈-Cycloalkyl, Halogen;
C₃-C₈-Cycloalkyl, das eine bis drei der folgenden Gruppen tragen kann: C₁-C₆-Alkyl, C₁-C₆-Halogenalkyl, C₁-C₄-Alkoxy, C₁-C₄-Halogenalkoxy, Halogen oder Nitro;
C₃-C₆-Alkenyl oder C₃-C₆-Alkinyl, die bis zu dreimal durch Halogen substituiert sein können, bedeutet und
R², und R³ gleich oder verschieden sind und für Halogen, Cyano, C₁-C₆-Alkyl, welches durch ein bis fünf Halogenatome und/oder einen bis zwei der folgenden Reste substituiert sein kann: C₁-C₄-Alkoxy, C₁-C₄-Halogenalkoxy, C₁-C₄-Alkylthio, C₁-C₄-Halogenalkylthio, C₃-C₆-Cycloalkyl oder Cyano;
für Benzyl, das bis zu dreimal durch C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro substituiert sein kann;
für C₃-C₈-Cycloalkyl, das ein- bis dreimal durch C₁-C₄-Alkyl oder Halogen substituiert sein kann;
für C₂-C₆-Alkenyl, welches bis zu dreimal durch Halogen und/oder einmal durch C₁-C₃-Alkoxy oder durch Phenyl, das eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro, substituiert sein kann;
für C₂-C₆-Alkinyl, welches bis zu dreimal durch Halogen oder C₁-C₃-Alkoxy und/oder einmal durch Phenyl, das eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro, substituiert sein kann;
für C₁-C₄-Alkoxy, C₁-C₄-Alkylthio, C₁-C₄-Halogenalkoxy, C₁-C₄-Halogenalkylthio, C₂-C₅-Alkenyloxy, C₂-C₅-Alkinyloxy, C₁-C₄-Alkylsulfinyl, C₁-C₄-Alkylsulfonyl, C₁-C₄-Halogenalkylsulfonyl;
für Phenoxy oder Phenylthio, welche bis zu dreimal durch C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro substituiert sein können;
für einen 5- oder 6-gliedrigen heterocyclischen Rest mit einem oder zwei Heteroatomen, ausgewählt aus der Gruppe Sauerstoff, Schwefel und Stickstoff, der ein bis zwei Substituenten der folgenden Gruppen tragen kann: C₁-C₃-Alkyl, Halogen, C₁-C₃-Alkoxy oder C₂-C₄-Alkoxycarbonyl;
oder
für Phenyl, welches eine bis drei der folgenden Gruppen tragen kann: C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Halogen, Nitro oder Cyano
stehen, mit der Maßgabe daß mindestens einer der Reste R² und R³ für Fluor steht;
R⁴ Wasserstoff bedeutet oder die für R² und R³ genannte Bedeutung haben kann;
sowie deren umweltverträgliche Salze.

Weiterhin betrifft die Erfindung Verfahren zur Herstellung der Verbindungen I und ihre Verwendung zur Bekämpfung unerwünschten Pflanzenwuchses. Gegenstand der Erfindung sind ferner neue Pyridin-2,3-dicarbonsäureester sowie Pyridin-2,3-dicarbonsäureanhydride, jeweils insbesondere fluorsubstituiert, die als Zwischenprodukte zur Herstellung der Verbindungen I dienen.

N-substituierte Pyridindicarbonsäureimide bzw. deren Derivate sind bekannt. In EP-A 128 006 werden u.a. N-Cycloalkylen-pyridindicarbonsäureimide und deren Verwendung als Bodenfungizide beschrieben.

In US-A 3 539 568 wird ein Verfahren zur Herstellung von 2,3- und 3,4-Pyridindicarbonsäureimiden und deren Umsetzung zu isomeren Dicarbonsäureamiden beschrieben, die als Zwischenprodukte für herbizide Pyrimidindione verwendet werden können.

Aus US-A 4 261 730 sind 3-Carboxy-pyridin-2-N-(aryl)-carbonsäureamide bekannt sowie Phthalamidsäuren mit wachstumsregulierender Wirkung.

In US-A 4 658 030 wird ein Verfahren zur Herstellung herbizider 2-(Imidazolin-2-yl)-nikotinsäuren auf Basis von 3-Carboxy-pyridin-2-(N-2-carbamido-3-methyl-2-butyl)-carbonsäureamiden offenbart.

Aus Helv. Chim. Acta 1988, Bd. 71, S. 486 und 493 ist ein Cycloadditions-Verfahren zur Herstellung von Pyridin-2,3-dicarboximiden bekannt.

J 5 7085-386 beschreibt speziell substituierte Pyridin-2,3-dicarbonsäureimide mit Antitumorwirkung.

Herbizid wirksame Pyridin-2,3-dicarbonsäureimide sind aus EP-A 422 456 sowie aus GB-A-2 174 395 und EP-A-41 623 bekannt.

Hinsichtlich der neuen Zwischenprodukte ist folgender Stand der Technik heranzuziehen: In J. Med. Chem. 17, 1065 wird 5-Chlor-pyridin-2,3-dicarbonsäureimid offenbart. Halogensubstitutierte Anhydride wie 5-Halogen-pyridin-2,3-dicarbonsäureanhydrid, 5-Chlor- oder Perfluor-pyridin-2,3-dicarbonsäureanhydrid sind aus Chemical Abstracts, Bd. 84, 135440 und Bd. 55, 23525 sowie Chem. Ber.56, 2457 bekannt. Einzelne Pyridin-2,3-dicarbonsäureester bzw. -chloride sind in Chemical Abstracts, Bd. 206, 213955, DE-A 36 34 975, EP-A 227 932 und EP-A 299 362 beschrieben.

Der Erfindung lag nun die Aufgabe zugrunde, Pyridin-2,3-dicarbonsäureimide mit verbesserter biologischer Wirksamkeit insbesondere besserer Selektivität bzw. hoher herbizide Aktivität bereitzustellen.

Demgemäß wurden die eingangs definierten Pyridin-2,3-dicarbonsäureimide gefunden.

Bevorzugt sind Pyridin-2,3-dicarbonsäureimide der Formel I, in der R¹ Wasserstoff, C₁-C₆-Alkyl, C₃-C₆-Alkenyl, C₃-C₆-Alkinyl, 1-(Cyclopropyl)-C₁-C₃-alkyl, 1-C₁-C₂-Alkyl-cyclopropyl oder C₃-C₆-Cycloalkyl bedeutet und zwei der drei Reste im Pyridinring R², R³ oder R⁴ unabhängig voneinander C₁-C₄-Alkyl, welches durch ein bis fünf Halogenatome substituiert sein kann, C₁-C₄-Alkoxy, Halogen oder Cyano bedeuten, und einer der Reste R², und R³ für Fluor steht.

Der Substituent am Imidstickstoff R¹ stellt vorzugsweise einen Alkylrest, insbesondere einen verzweigten Alkylrest wie i-Propyl, sek.-Butyl oder tert.-Butyl dar oder einen 1-(Cycloalkyl)-C₁-C₃-alkylrest wie 1-(Cyclopropyl)-methyl, 1-(Cyclopropyl)-ethyl oder 1-(Cyclopropyl)-propyl.

Gegenstand der Erfindung sind ferner neue Zwischenprodukte wie Anhydride und fluorsubstituierte Pyridin-2,3-dicarbonsäureester. Hier sind folgende Verbindungen zu nennen:

Pyridin-2,3-dicarbonsäureanhydride der Formel IIa' 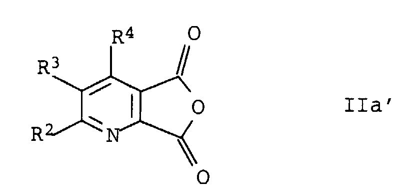 in der einer der Reste R² und R³ Fluor bedeutet und die übrigen Reste für Wasserstoff, Fluor, Chlor, C₁-C₃-Alkyl,C₁-C₃-Alkoxy, Trifluormethoxy, Chlordifluormethoxy, Methylthio, Trifluormethylthio, Chlordifluormethylthio oder Methylsulfonyl stehen, ausgenommen 5-Fluor- und 4,5,6-Trifluorpyridin-2,3-dicarbonsäureanhydrid.

Besonders interessante Zwischenprodukte sind 6-Fluor-pyridin-2,3-dicarbonsäureanhydrid und 5,6-Difluor-pyridin-2,3-dicarbonsäureanhydrid.

Ferner sind bevorzugte erfindungsgemäße Verbindungen Pyridin-2,3-dicarbonsäureanhydride der Formel IIa' 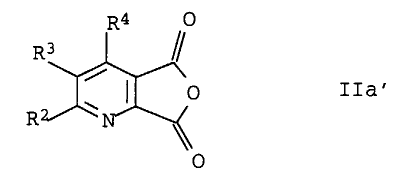 in der R⁴ Wasserstoff bedeutet, R³ für Chlor, C₁-C₃-Alkoxy, Trifluormethyl oder Cyano steht und R² für Fluor steht.

Erfindungsgemäße Zwischenprodukte auf der Dialkylesterstufe sind 4,5 und/oder 6-Fluor-pyridin-2,3-dicarbonsäure-dialkylester der Formeln Va', Va'' und Va''': 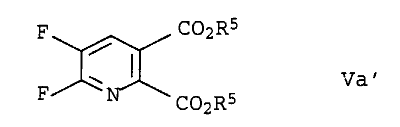 in der R⁵ einen C₁-C₄-Alkyl-, C₃-C₅-Alkenyl- oder C₃-C₅-Alkinylrest, ggf. substituiert durch Halogen, Phenyl oder C₁-C₄-Alkoxy darstellt, 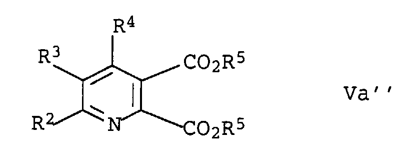 in der einer der Reste R² oder R³ Fluor bedeutet und die übrigen Reste R², R³ oder R⁴ für Wasserstoff, Chlor, C₁-C₃-Alkyl, C₁-C₃-Alkoxy, Trifluormethoxy, Chlordifluormethoxy, Methylthio, Trifluormethylthio, Chlordifluormethylthio oder Methylsulfonyl stehen und R⁵ einen C₂-C₄-Alkyl-, C₃-C₅-Alkenyl- oder C₃-C₅ Alkinylrest, ggf. substituiert durch Halogen, Phenyl oder C₁-C₄-Alkoxy darstellt sowie 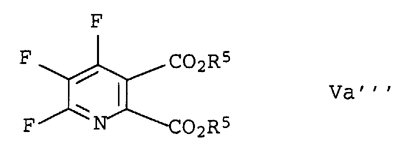 in der R⁵ einen C₂-C₄-Alkyl-, C₃-C₅-Alkenyl- oder C₃-C₅ Alkinylrest, ggf. substituiert durch Halogen, Phenyl oder C₁-C₄-Alkoxy darstellt.

Die Pyridin-2,3-dicarbonsäureimide der Formel I können mit anorganischen Säuren oder mit Alkylhalogeniden Additionssalze bilden oder sie können, sofern einer der Substituenten saure Eigenschaften hat, mit anorganischen und organischen Basen zu Salzen umgesetzt werden. Die entsprechenden Salze gehören ebenfalls zur Erfindung.

Als basische Salze kommen z.B. diejenigen der Alkalimetalle, vorzugsweise die Natrium- und Kaliumsalze, die der Erdalkalimetalle, vorzugsweise Calcium-, Magnesium-, und Bariumsalze und die der Übergangsmetalle, vorzugsweise Mangan-, Kupfer-, Zink- und Eisensalze sowie die Ammoniumsalze, die ein bis drei C₁-C₄-Alkyl-, Hydroxy-C₁-C₄-alkylsubstituenten und/oder einen Phenyl- oder Benzylsubstituenten tragen können, vorzugsweise Diisopropylammonium-, Tetramethylammonium-, Tetrabutylammonium-, Trimethylbenzylammonium- und Trimethyl-(2-hydroxy-ethyl)-ammoniumsalze, die Phosphoniumsalze, die Sulfoniumsalze, vorzugsweise Tri-(C₁-C₄-)alkylsulfoniumsalze, und die Sulfoxoniumsalze, vorzugsweise Tri-(C₁-C₄-)alkylsulfoxoniumsalze in Betracht.

Sofern das Substitutionsmuster am Pyridin-2,3-dicarbonsäureimid I zu optisch aktiven Verbindungen führt, so gehören zum Gegenstand der Erfindung neben den Racematen auch die (+) und (-) Enantiomeren.

Die Pyridin-2,3-dicarbonsäureimide der Formel I sind auf verschiedenen Wegen herstellbar, die in EP-A 422 456 beschrieben sind.

Erfindungsgemäß erhält man Pyridinderivate der Formel I durch Umsetzung von Pyridindicarbonsäureanhydriden II 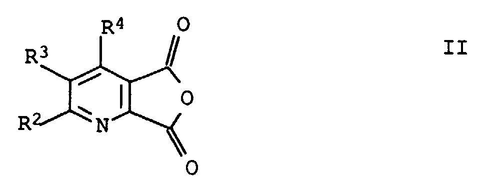 in einem inerten organischen Lösungsmittel mit einem primären Amin III (bevorzugt in ungefähr stöchiometrischen Mengen)

H₂N-R¹ III

zu einem Pyridindicarbonsäurehalbamid IV 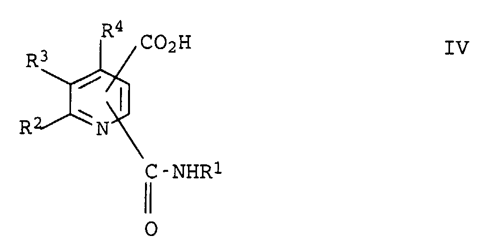 und dessen Cyclisierung mit wasserabspaltenden Mitteln zu I.

Außerdem wurden chemisch eigenartige Verfahren zur Herstellung der Verbindungen Ia gefunden, 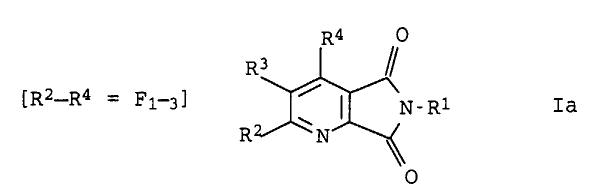 in denen R² bis R⁴ die vorgenannte Bedeutung besitzen, wobei mindestens einer der Reste R², und R³ für Fluor steht. Im Vergleich zum Stand der Technik lassen sich die Pyridinderivate Ia in hoher Ausbeute und Reinheit herstellen, wenn man Pyridinderivate Ib, 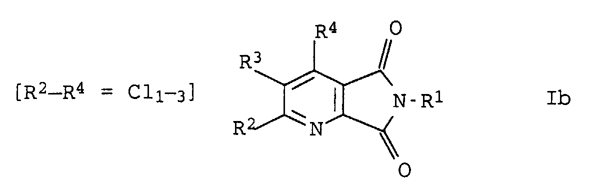 in denen R² bis R⁴ die vorgenannte Bedeutung besitzen, wobei mindestens einer der Reste R² und R³ für Chlor steht, einem Halogenaustausch mit Kaliumfluorid unterzieht.

Im Rahmen der Herstellung der erfindungsgemäßen Verbindungen wurde weiterhin gefunden, daß man Pyridinderivate der Formel Ia ebenfalls vorteilhaft erhält, wenn man von Pyridindicarbonsäureanhydriden der Formel IIb ausgeht, 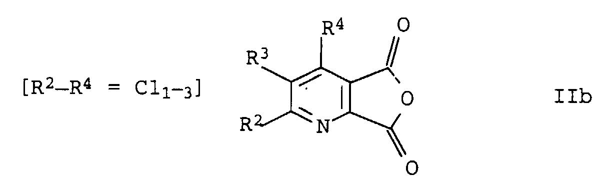 in der R² bis R⁴ die vorgenannte Bedeutung besitzen, wobei mindestens einer der Reste R² und R⁴ für Chlor steht, diese mit Kaliumfluorid einem Halogenaustausch unterzieht zu Pyridindicarbonsäureanhydriden IIa 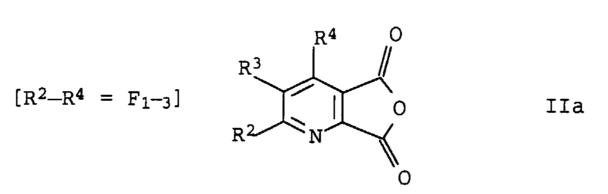 in denen R² bis R⁴ die vorgenannte Bedeutung besitzen, wobei mindestens einer der Reste R², und R⁴ für Fluor steht, und diese dann mit einem substituierten Amin, wie beschrieben, in die Pyridinderivate Ia überführt.

Die Darstellung der Pyridindicarbonsäurehalbamide IV wird zweckmäßigerweise so durchgeführt, daß man das Anhydrid II in einem inerten Lösungsmittel vorlegt und etwa molare Mengen eines Amins III, gegebenenfalls ebenfalls gelöst in einem inerten Lösungsmittel, zutropft. Nach beendeter Reaktion wird das Reaktionsprodukt abgesaugt oder durch Einengen des verwendeten Lösungsmittels isoliert, wobei man die Halbamide IV erhält. 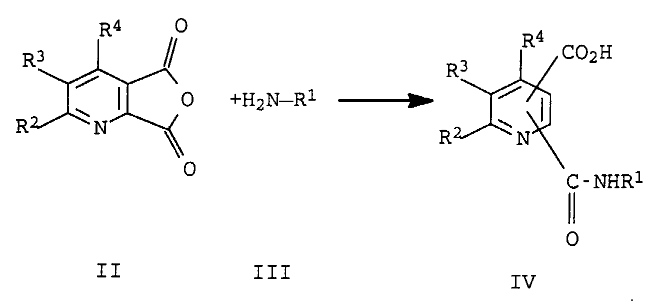 Zweckmäßigerweise verwendet man für diese Umsetzungen Lösungsmittel wie Halogenkohlenwasserstoffe, z.B. Tetrachlorethan, Methylenchlorid, Chloroform, Chlorbenzol und 1,2-Dichlorbenzol; Ether z.B. Diethylether, Methyl-tert.-butylether, Dimethoxyethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromaten, z.B. Benzol, Toluol, Xylol, Pyridin und Chinolin; Ketone, z.B. Acton, Methylethylketon oder entsprechende Gemische.

Die Umsetzung kann bei Temperaturen von -10°C bis zur Rückflußtemperatur des jeweiligen Lösungsmittels bzw. -gemisches, vorzugsweise bei -20 bis 120°C, durchgeführt werden.

Die molaren Verhältnisse, in denen die benötigten Ausgangsverbindungen miteinander umgesetzt werden, betragen vorzugsweise 5 0,9:1 bis 3:1 für das Verhältnis von Amin III zu Anhydrid II. Die Konzentration der Edukte im Lösungsmittel beträgt z.B. 0,1 bis 5 mol/l, bevorzugt 0,2 bis 2 mol/l.

Die für dieses Verfahren als Ausgangsmaterialien benötigten Pyridindicarbonsäuren bzw. -ester und -anhydride sind kommerziell erhältlich, literaturbekannt oder können nach allgemein bekannten Methoden hergestellt werden. Eine Übersicht findet sich in Beilstein H 22, 150-160, E I 531-536, E II 104-111, H 27, 261, E I 319, E II 299, R. C. Elderfield, Heterocyclic Compounds, Vol. I, Chapt. 8, J. Wiley and Sons, N.Y., E. Klingberg "Pyridine and its Derivatives", Part 3, Chapt. X, in The Chemistry of Heterocyclic Compounds, 1962 Interscience Publishers sowie in EP-A 299 362 und EP-A 422 456.

Nach diesen sowie in EP-A 422 456 beschriebenen Methoden lassen sich auch neue 4- und 6-halogen- bzw. 4,6-dihalogensubstituierte Pyridin-2,3-dicarbonsäureester durch Umsetzung von entsprechenden 4-H-, 6-H- bzw. 4-H-6-H-Pyridin-2,3-dicarbonsäureester-N-oxiden mit Phosphorylhalogeniden herstellen. Hierbei wandert das Halogen im Zuge der Umlagerungsreaktion zunächst in die freie 6-Position; ist diese besetzt, so tritt das Halogen in die 4-Position ein.

Die Cyclisierung der Halbamide IV erfolgt durch Wasserentzug mit üblichen wasserabspaltenden Mitteln, beispielsweise Acetanhydrid oder anorganischen Säurehalogeniden, wie Thionylchlorid, Phosgen, Phosphortri- oder pentachlorid, zu den Pyridinderivaten der Formel I. Die Reaktion wird zweckmäßigerweise so durchgeführt, daß man die Carbonsäureamide in einem inerten organischen Lösungsmittel vorlegt und das wasserabspaltende Mittel, gegebenenfalls ebenfalls gelöst in einem inerten Lösungsmittel, z.B. Dimethylformamid (DMF), zutropft. Das Gemisch kann wie üblich aufgearbeitet werden, beispielsweise durch Hydrolyse mit Wasser und Absaugen oder Extraktion des Produktes mit einem organischen Lösungsmittel und Einengen des organischen Lösungsmittels: 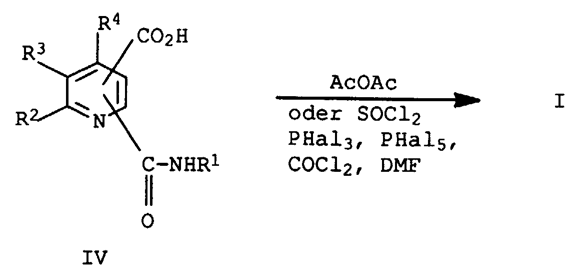 Zweckmäßigerweise verwendet man für diese Umsetzungen Lösungsmittel wie Halogenkohlenwasserstoffe, z.B. Tetrachlorethan, Methylenchlorid, Chloroform, Chlorbenzol und 1,2-Dichlorbenzol; Ether z.B. Diethylether, Methyl-tert.-butylether, Dimethoxyethan, Diethylenglykoldimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolidin-2-on; Aromaten, z.B. Benzol, Toluol, Xylol, Pyridin und Chinolin; Ketone, z.B. Aceton, Methylethylketon oder entsprechende Gemische.

Die Umsetzung kann bei Temperaturen von -10°C bis zur Rückflußtemperatur des jeweiligen Lösungsmittels, vorzugsweise bei 0 bis 150°C, durchgeführt werden.

Die molaren Verhältnisse, in denen die Ausgangsverbindungen miteinander umgesetzt werden, betragen im allgemeinen 0,9:1 bis 5:1 wasserabspaltendes Mittel zu Säureamid.

Die Konzentration der Edukte im Lösungsmittel(gemisch) beträgt im allgemeinen 0,1 bis 5 mol/l, bevorzugt 0,2 bis 2 mol/l.

Ein besonders vorteilhaftes Verfahren zur Herstellung von Verbindungen Ia, in denen mindestens einer der Reste R² bis R⁴ für Fluor steht, oder 2 bzw. 3 Reste Fluor bedeuten, besteht in dem Halogenaustausch eines entsprechend chlorsubstituierten Pyridinderivats Ib mit Kaliumfluorid.

Die Reaktion wird bevorzugt so durchgeführt, daß man ein Pyridinderivat Ib in Gegenwart eines polaren Lösungsmittels mit Kaliumfluorid bei Temperaturen zwischen 70 bis 250°C, vorzugsweise 80 bis 200°C, behandelt.

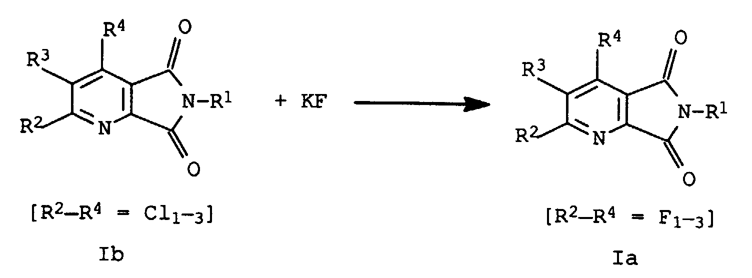

Als Lösungsmittel verwendet man für diese Umsetzungen Nitrile wie Acetonitril, Propionitril, Butyronitril, Isobutyronitril, Glykolether wie Dimethylglykolether. Diethylglykolether, Diethylenglykoldimethylether, Carbonsäureamide wie DMF, N-Methylpyrrolidon, Harnstoffe wie Tetraethylharnstoffe, Tetrabutylharnstoff, Dimethylenharnstoff, Dimethylpropylenharnstoff, Sulfoxide wie Dimethylulfoxid und vorzugsweise Sulfone wie Dimethylsulfon, Diethylsulfon, Tetramethylensulfon (Sulfolan) oder Pentamethylensulfon. Auch die Durchführung in der Schmelze ohne Zusatz eines Lösungsmittels ist erfindungsgemäß möglich.

Der Halogenaustausch verläuft bereits ohne Katalysator mit hoher Geschwindigkeit. Er kann jedoch durch Zugabe eines Katalysators, z.B. eines Kronenethers oder Cryptanden beschleunigt werden. Diese sind organische Komplexliganden, die insbesondere gut zur Bindung von Alkali dienen. Die Cryptanden liefern eine dreidimensionale Umhüllung. Bezüglich Herstellung dieser Stoffe, siehe "Kontakte" (1977), Seiten 11 bis 31 und 36 bis 48. Als Katalysator sind Kronenether bevorzugt, von denen z.B. die folgenden Verbindungen genannt sein sollen: 12-Krone-4, 14-Krone-4, Dibenzo-14-krone-4, 18-Krone-5, 18-Krone-6, Dibenzo-18-krone-6 oder Aza-18-krone-6.

Diese Katalysatoren werden zweckmäßig in einer Menge von 0,05 bis 5, insbesondere 0,1 bis 2 Molprozent je Mol Ausgangsstoff Ib eingesetzt.

Die molaren Verhältnisse, in denen die Ausgangsverbindungen miteinander umgesetzt werden, betragen 0,9:1 bis 2,0:1, vorzugsweise 1,1:1 bis 1,3:1 für das Verhältnis von Kaliumfluorid zu Pyridinderivat Ib. Die Konzentration der Edukte im Lösungsmittel beträgt 0,1 bis 5 mol/l, bevorzugt 0,2 bis 2 mol/l.

Besonders vorteilhaft lassen sich die Verbindungen Ia herstellen, wenn man vor dem eigentlichen Halogenaustausch das Pyridinderivat Ib beispielsweise in Gegenwart eines aliphatischen Sulfons bei Temperaturen bis 150°C, zweckmäßig zwischen 50 und 120°C, insbesondere 70 bis 100°C mit 0,1 bis 0,4, zweckmäßig 0,15 bis 0,3 mol eines Säurechlorids der schwefligen Säure oder Kohlensäure behandelt und das Reaktionsgemisch dann bei Temperaturen von 70 bis 250°C, vorzugsweise 80 bis 200°C mit Kaliumfluorid umsetzt.

Als Katalysatoren sind für diese Verfahrensstufe z.B. N,N-disubstituierte Carbonamide wie DMF, N,N-Dimethylacetamid oder N,N-Diisopropylacetamid geeignet. Der Katalysator wird zweckmäßig in einer Menge von 0,2 bis 2 Gewichtsprozent, bezogen auf das Säurechlorid, eingesetzt.

Bei der Umsetzung mit dem Säurechlorid erhitzt man zweckmäßig so lange, bis keine Gasentwicklung mehr erfolgt. Es empfiehlt sich, überschüssiges Säurechlorid z.B. durch Einblasen eines Inertgases, wie Stickstoff, oder durch Anlegen von Vakuum zu entfernen.

Zu diesem Gemisch gibt man dann das Kaliumfluorid, das zweckmäßig vorher getrocknet wurde und hält die durch Verrühren erhaltene Mischung 1 bis 10 Stunden auf Reaktionstemperatur.

Als Fluoridsalze kommen erfindungsgemäß neben Kaliumfluorid auch Tetraalkyl-(C₁-C₁₃)-anmoniumfluorid sowie entsprechende Mischungen untereinander oder mit Cäsiumfluorid oder Rubidiumfluorid in Frage, wobei diese Mischungen mit Cäsiumfluorid nicht mehr als 50 Gew.-% an Cäsiumfluorid enthalten. Bevorzugt werden Fluoridmischungen verwendet, die zumindest 75 Gew.-% Kaliumfluorid enthalten; insbesondere bestehen derartige Mischungen aus wenigstens 90 Gew.-% Kaliumfluorid und höchstens 10 Gew.-% Cäsiumfluorid oder aus 60 Gew.-% Kaliumfluorid und 40 Gew.-% Rubidiumfluorid. In einer weiteren bevorzugten Ausgestaltungsform kommt als Fluoridsalz nur Kaliumfluorid zum Einsatz.

Als Phasentransferkatalysatoren können quartäre Ammonium- oder Phosphoniumsalze verwendet werden. An geeigneten Verbindungen seien folgende genannt: Tetraalkyl-(C₁-C₁₈)-ammoniumchloride, -bromide oder -fluoride, Tetraalkyl-(C₁-C₁₈)-phosphoniumchloride oder -bromide, Tetraphenylphosphoniumchlorid oder -bromid, (Phenyl)ₘ(alkyl-(C₁-C₁₈))ₙ-phosphoniumchloride oder -bromide, wobei m = 1 bis 3, n = 3 bis 1 und m + n = 4 ist. Auch Gemische dieser Salze sind einsetzbar. Bei geeigneter Wahl des Katalysators für die jeweilige umzusetzende Verbindung, was durch einige Routineversuche leicht zu ermitteln ist, erhält man deutlich bessere Raumleistungen und Ausbeuten als nach dem in der EP-OS 146 924 beschriebenen Verfahren; außerdem sind die Apparate-Totzeiten sowei die gesamten Apparatekosten nach dem erfindungsgemäßen Verfahren wesentlich günstiger.

Die Menge an Phasentransferkatalysator beträgt im allgemeinen bis zu 20 Gew.-%, bevorzugt zwischen 3 und 15 Gew.-% und besonders bevorzugt zwischen 3 bis 8 Gew.-%, bezogen auf die eingesetzte Menge an Fluoridsalz.

Als Phasentransferkatalysatoren können auch Oligo- oder Polyalkylenglykoldimethylether verwendet werden, wobei der Alkylenrest 2 bis 6 C-Atome, vorzugsweise 2 und/oder 3 C-Atome enthält, also vorzugsweise den Ethylen- und/oder den Propylenrest und insbesondere nur den Ethylenrest darstellt. Die Zahl der O-ethylen(Glykol)einheiten (-O-CH₂-CH₂-) und/oder der 0-propyleneinheiten in diesen Verbindungen kann von n = 4 (z.B. Tetraethylenglykoldimethylether) bis etwa n - 150 betragen; bevorzugt werden jedoch Ether eingesetzt, deren Polymerisationsgrad zwischen n = 4 und n - 25 beträgt. Im Falle von Alkylenresten mit mehr als 3 C-Atomen liegt n im allgemeinen nicht höher als 6. Die Einsatzmenge dieser Ether, insbesondere Glykolether liegt zumeist zwischen etwa 0,5 Gew.-% und etwa 200 Gew.-%, bevorzugt zwischen etwa 5 und etwa 100 Gew.-% und besonders bevorzugt zwischen etwa 10 und etwa 50 Gew.-%, bezogen auf die Menge des eingesetzten Fluoridsalzes. Der besondere Vorteil bei der Verwendung dieser Verbindungen liegt darin, daß meist der Einsatzmenge entsprechend weniger Lösungsmittel verwendet werden kann, da die Glykolether bei der Reaktionstemperatur im allgemeinen flüssig wird. Auch Gemische dieser Ether untereinander als auch Gemische dieser Ether (einzeln oder im Gemisch) mit den quartären Ammonium- oder Phosphoniumsalzen, vorzugsweise Glykolether mit quartären Phosphoniumsalzen, können eingesetzt werden.

Verwendet man als Fluoridsalz Tetraalkyl-(C₁-C₁₈)-ammoniumfluoride, so ist der Zusatz eines weiteren Phasentransferkatalysators nicht erforderlich, da das Fluoridsalz selbst einen solchen darstellt, der damit also in stöchiometrischen und größeren Mengen eingesetzt werden kann.

Die Verwendung von sprühgetrocknetem Alkalimetallfluorid im erfindungsgemäßen Verfahren verkürzt dabei zwar zum Teil die Reaktionszeiten, ist aber nicht unbedingt notwendig. Ebenso kann unter Zusatz von säurebindenden Mitteln, wie Alkali- und Erdalkalimetallcarbonaten oder basisch wirkenden Oxiden, wie zum Beispiel Magnesiumoxid, oder entsprechenden Gemischen, gearbeitet werden. Besonders bevorzugt ist hierbei Kaliumcarbonat, das in Anteilen von etwa 1 bis etwa 10 Gew.-%, bevorzugt von etwa 4 bis etwa 6 Gew.-%, bezogen auf die Fluoridsalz-Menge, verwendet wird.

Die säurebindenden Mittel sind für den Reaktionsverlauf im allgemeinen nicht wesentlich. In einigen Fällen wird die Reaktionsgeschwindigkeit durch die Bildung von Fuorwasserstoff während der Reaktion erheblich verringert. In diesen Fällen ist es günstig, besonders auch zur Vermeidung von Apparatekorrosion, unter Anwesenheit von derartigen Säurefängern zu arbeiten. Der Einsatz dieser Verbindungen bei Fraktionierung des Reaktionsgemisches oder des Rohproduktes kann aus Gründen der Korrosion in der Fraktionieranlage wünschenswert sein, wobei Magnesiumoxid hierbei besonders bevorzugt ist. Zu diesem Zweck werden der Fraktionierblase bis etwa 10 Gew.-% an Säurefänger zugesetzt, bevorzugt zwischen etwa 3 und 8 Gew.-%, bezogen auf die Gesamtmenge an eingesetzten Destillationssumpf.

Nach der Umsetzung mit Alkalifluorid arbeitet man auf an sich übliche Weise, z.B. durch Filtration, Waschen des Festgutes, Destillation von Filtrat und Waschfiltraten, auf. Im Falle von wassermischbaren Lösungsmitteln kann man die Pyridinderivate Ia auch durch Zugabe von Wasser ausfällen und wie beschrieben aufarbeiten.

Das Verfahren zur Herstellung der Verbindungen IIa in denen mindestens einer der Reste R² und R³ für Fluor steht, besteht in dem Halogenaustausch eines Pyridindicarbonsäureanhydrids IIb mit Kaliumfluorid und wird auf gleiche Weise wie die oben beschriebene Umsetzung der Pyridinderivate Ib durchgeführt. 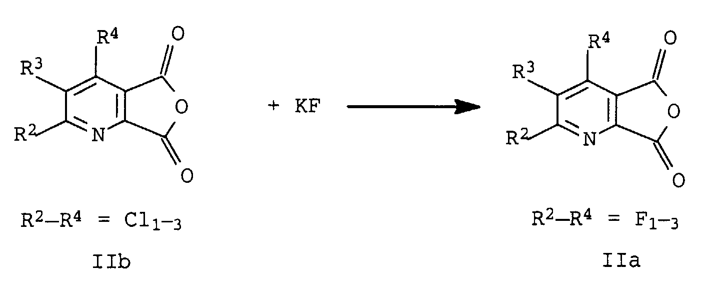 Statt auf der Stufe der Pyridindicarbonsäureanhydride IIb kann der Halogenaustausch auch auf der entsprechenden Diesterstufe Vb durchgeführt werden. Diese können anschließend entweder durch Hydrolyse und Cyclisierung in die Anhydride IIa umgewandelt oder direkt mit dem Amin III zu Carbonesterhalbamiden VI umgesetzt werden, die nach Hydrolyse zu IV zu den erfindungsgemäßen Verbindungen Ia cyclisiert werden können.

Entsprechend besteht ein Verfahren zur Herstellung von Pyridindicarbonsäureestern der Formel Va 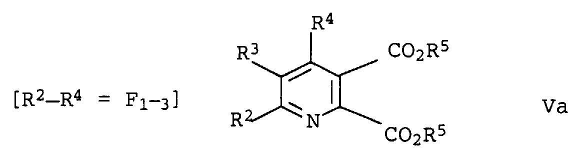 in der R² bis R⁴ die vorgenannte Bedeutung besitzen, wobei mindestens einer der Reste R² und R³ für Fluor steht und R⁵ einen ggf. substituierten Alkyl-, Alkenyl- oder Alkinylrest, z.B. Halogen-C₁-C₄-alkyl, Halogen-C₃-C₅-alkenyl, Halogen-C₃-C₅-alkinyl, C₁-C₄-Alkoxy-C₁-C₄-alkyl, C₁-C₄-Alkoxy-C₃-C₅-alkenyl, C₁-C₄-Alkoxy-C₃-C₅-alkinyl, Phenyl-C₁-C₄-alkyl, Phenyl-C₃-C₅-alkenyl, Phenyl-C₃-C₅-alkinyl, darstellt, in dem Halogenaustausch eines Pyridindicarbonsäureesters der Formel Vb, 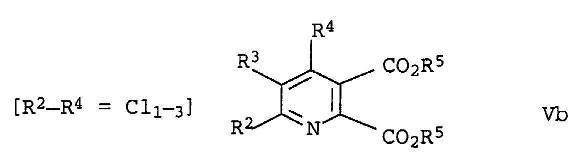 in der R² bis R⁵ die vorgenannte Bedeutung besitzen, wobei mindestens einer der Reste R² und R³ für Chlor steht, mit Kaliumfluorid.

Das Verfahren wird auf gleicher Weise wie die oben beschriebene Umsetzung der Pyridinderivate Ib durchgeführt. Daran schließen sich die oben aufgeführten Umwandlungen zu den Pyridinderivaten Ia an. 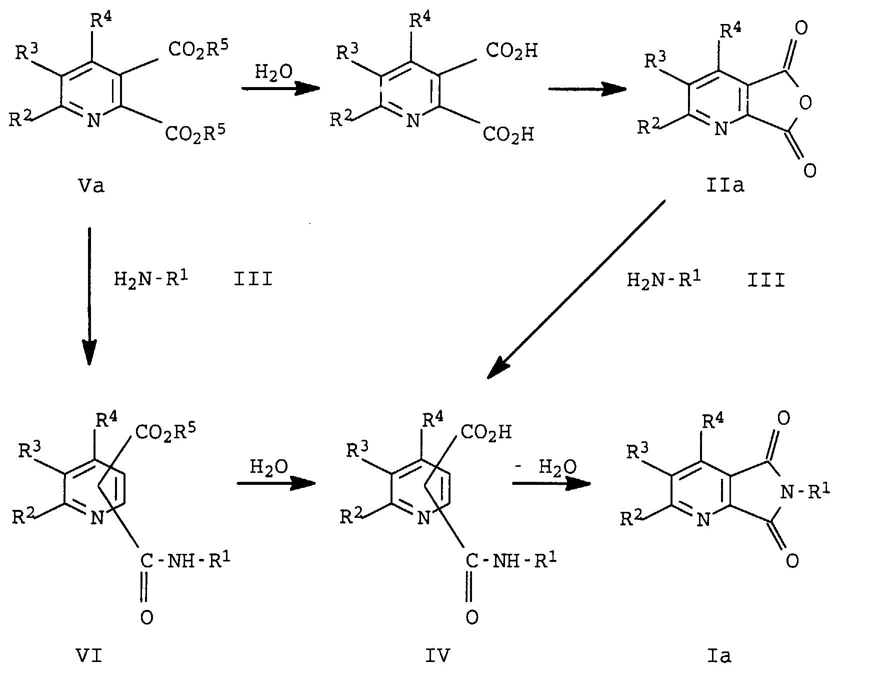 R² bis R⁴ = F₁₋₃

Die molaren Verhältnisse und die Durchführung lehnen sich an die Reaktionsbedingungen zur Darstellung von IV oder Ia an oder können in den einzelnen Verfahrensschritten nach allgemein literaturbekannten Methoden ausgeführt werden.

Im Vergleich zu dem Stand der Technik sind die neuen fluorsubstituierten Pyridin-2,3-dicarbonsäureanhydride, -ester und -imide auf besonders einfache Weise in hohen Ausbeuten zugänglich. Fluorsubstituierte Diester sind bisher nicht bekannt. In der Reihe der Anhydride wurden Herstellwege für das 5-Fluor- und das 4,5,6-Trifluor-pyridinderivat in J. Org. Chem. 26, 808 (1961) und J. Fluorine Chem. 7, 363 (1976) beschrieben. Nach beiden Methoden müssen erst auf umständliche Weise fluorsubstutuierte Chinoline aufgebaut und diese dann wieder oxydativ zerstört werden. Die Ausbeute der durch elektrochemische Oxydation gewonnenen 5-Fluorpyridin-2,3-dicarbonsäure beträgt nur 26 % und im Falle einer Salpetersäureoxydation des Heptafluorchinolins erhält man - als

Anhydrid isoliert - nur 5 % 4,5,6-Trifluor-pyridin-2,3-dicarbonsäureanhydrid neben 20 % 2,3,4,6,7-Pentafluor-5,8-dioxo-5,8-dihydro-chinolin und 12 % 3,4,5,6,7,8-Hexafluor-2-oxo-1,2-dihydrochinolin.

Im Hinblick auf die bestimmungsgemäße Verwendung der Verbindungen I kommen als Substituenten beispielsweise folgende Reste in Betracht:
R¹ Wasserstoff;
C₁-C₄-Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy;
C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, l-Ethyl-1-methylpropyl und l-Ethyl-2-methylpropyl, insbesondere Propyl, 1-Methylethyl und 1,1-Dimethylethyl, wobei die genannten Reste eine bis drei der folgenden Gruppen tragen können:
C₁-C₄-Alkoxy wie oben genannt, insbesondere Methoxy und Ethoxy;
C₁-C₄-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy;
C₁-C₄-Alkylthio wie Methylthio, Ethylthio, Propylthio, 1-Methylethylthio, n-Butylthio, 1-Methylpropylthio, 2-Methylpropylthio und 1,1-Dimethylethylthio, insbesondere Methylthio und Ethylthio;
C₁-C₄-Halogenalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio und Pentafluorethylthio;
(C₁-C₄-Dialkyl)amino wie Dimethylamino, Diethylamino, Dipropylamino, Diisopropylamino, Dibutylamino, Methylethylamino, insbesondere Dimethylamino und Methylethylamino;
C₃-C₈-Cycloalkyl wie Cyclopropyl, Cyclobutyl, Cyclopentyl, Cyclohexyl, Cycloheptyl und Cyclooctyl, insbesondere Cyclopropyl, Cyclopentyl und Cyclohexyl;
Halogen wie Fluor, Chlor, Brom, Jod, insbesondere Fluor und Chlor;
R¹ ferner C₃-C₈-Cycloalkyl wie oben genannt, insbesondere Cyclopropyl, Cyclobutyl; Cyclopentyl und Cyclohexyl, das eine bis drei der folgenden Gruppen tragen kann: C₁-C₆-Alkyl wie oben genannt, insbesondere Methyl, Ethyl und Isopropyl; Halogenalkyl wie oben für die homologen Halogenalkylthioreste genannt, insbesondere Trifluormethyl; C₁-C₄-Alkoxy wie oben genannt, insbesondere Methoxy und Ethoxy; C₁-C₄-Halogenalkoxy wie oben genannt, insbesondere Trifluormethoxy; Halogen wie oben genannt, insbesondere Fluor und Chlor;
C₃-C₆-Alkenyl wie 2-Propenyl, 2-Methylethenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 1-, 2-, 3-, 4- oder 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-pentenyl und Ethyl-2-methyl-2-pentenyl, insbesondere Ethenyl, 2-Propenyl, 1-Methylethenyl, 2-Butenyl, 3-Butenyl, 1-Methylpropyl, 1-Methyl-2-propenyl, 2-Methyl-2-propenyl, welches ein- bis dreimal durch Halogen, insbesondere Fluor und Chlor substituiert sein kann;
R¹ ferner C₃-C₆-Alkinyl wie Propargyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und l-Ethyl-1-methyl-2-propinyl, insbesondere 1-Methyl-2-propinyl und 1,1-Dimethyl-2-propinyl, welches ein- bis dreimal durch Halogen wie vorstehend genannt, insbesondere Fluor und Chlor und/oder einmal durch Phenyl substituiert sein kann;
R² und R³ Halogen wie unter R¹ genannt, insbesondere Fluor und Chlor; Cyano; C₁-C₆-Alkyl, wie unter R¹ genannt, insbesondere Methyl, Ethyl, Propyl, 1-Methylethyl und 1,1-Dimethylethyl, welches ein bis fünf Halogenatome, wie unter R¹ genannt, insbesondere Fluor und Chlor, und/oder einen oder zwei der folgenden Reste tragen kann: C₁-C₄-Alkoxy wie unter R¹ genannt, insbesondere Methoxy, Ethoxy, 1-Methylethoxy und 1,1-Dimethylethoxy; C₁-C₄-Halogenalkoxy wie unter R¹ genannt, insbesondere Halogenmethoxy wie Difluormethoxy und Trifluormethoxy; C₁-C₄-Alkylthio wie unter R¹ genannt, insbesondere Methylthio und Ethylthio; C₁-C₄-Halogenalkylthio, wie unter R¹ genannt, insbesondere Difluormethylthio und Trifluormethylthio;
C₃-C₆-Cycloalkyl wie unter R¹ genannt, insbesondere Cyclopropyl;
Benzyl, das ein bis dreimal durch C₁-C₄-Alkyl wie unter R¹ genannt, insbesondere Methyl, Ethyl und 1-Methylethyl; C₁-C₄-Halogenalkyl wie unter R¹ genannt, insbesondere Trifluormethyl und Chlordifluormethyl; C₁-C₄-Alkoxy wie unter R¹ genannt, insbesondere Methoxy und Ethoxy; C₁-C₄-Halogenalkoxy wie unter R¹ genannt, insbesondere Trifluormethoxy, Trichlormethoxy und Pentafluorethoxy; C₁-C₄-Alkylthio wie unter R¹ genannt, insbesondere Methylthio und Ethylthio; C₁-C₄-Halgenalkylthio wie unter R¹ genannt, insbesondere Difluormethylthio, Trifluormethylthio und Pentafluormethylthio; Halogen insbesondere Fluor und Chlor; Cyano oder Nitro substituiert sein kann;
C₃-C₈-Cycloalkyl, wie unter R¹ genannt, insbesondere Cyclopropyl, Cyclopentyl und Cyclohexyl; das ein- bis dreimal durch C₁-C₄-Alkyl wie unter R¹ genannt, insbesondere
Methyl und Ethyl; oder Halogen wie unter R¹ genannt, insbesondere Fluor und Chlor, substituiert sein kann;
C₂-C₆-Alkenyl wie unter R¹ genannt, ferner 1-Ethenyl, 1-Propenyl, 1-Methyl-1-propenyl, 2-Methyl-1-propenyl, 1-Pentenyl, 1-Methyl-1-butenyl, 2-Methyl-1-butenyl, 1-Ethyl-1-propenyl, 1-Methyl-1-pentenyl, 2-Methyl-1-pentenyl, 3-Methyl-1-pentenyl, 4-Methyl-1-pentenyl, 1,2-Dimethyl-1-butenyl, 1,3-Dimethyl-1-butenyl, 2,3-Dimethyl-1-butenyl, 3,3-Dimethyl-1-butenyl, Ethyl-1-butenyl, 2-Ethyl-1-butenyl, 1-Ethyl-2-methyl-1-pentenyl, insbesondere Ethenyl, 1-Propenyl, 2-Propenyl, 1-Methylethenyl, 1-Butenyl, 2-Butenyl, 3-Butenyl, 1-Methylpropenyl, 1-Methyl-2-propenyl, 2-Methyl-1-propenyl, 2-Methyl-2-propenyl, welches ein bis dreimal durch Halogen wie unter R¹ genannt, insbesondere Fluor und Chlor; oder C₁-C₃-Alkoxy wie unter R¹ genannt, insbesondere Methoxy und Ethoxy; und/oder einmal durch Phenyl substituiert sein kann, wobei der Phenylrest seinerseits eine bis drei der folgenden Gruppen tragen kann: Alkyl wie unter R¹ genannt, insbesondere Methyl, Ethyl und 1-Methylethyl; Halogenalkyl wie unter R¹ genannt, insbesondere Trifluormethyl und Chlordifluormethyl; Alkoxy wie unter R¹ genannt, insbesondere Methoxy und Ethoxy; Halogenalkoxy wie unter R¹ genannt, insbesondere Trifluormethoxy, Trichlormethoxy und Pentafluorethoxy; Alkylthio wie unter R¹ genannt, insbesondere Methylthio und Ethylthio; Halogenalkylthio wie unter R¹ genannt, insbesondere Difluormethylthio, Trifluormethylthio und Pentafluormethylthio; Halogen wie unter R¹ genannt, insbesondere Fluor und Chlor; Cyano oder Nitro;
C₂-C₆-Alkinyl, wie unter R¹ genannt, ferner Ethinyl, 1-Propinyl, 1-Butinyl, 1-Pentinyl, 1-Hexinyl, 3-Methyl-1-pentinyl, 4-Methyl-1-pentinyl, insbesondere Ethinyl, 1-Propinyl und Propargyl, welches ein- bis dreimal durch Halogen wie vorstehend genannt, insbesondere Fluor und Chlor; oder Alkoxy wie vorstehend genannt, insbesondere Methoxy und Ethoxy; und/oder einmal durch Phenyl substituiert sein kann, wobei der Phenylrest seinerseits eine bis drei der folgenden Gruppen tragen kann: Alkyl wie vorstehend genannt, insbesondere Methyl, Ethyl und 1-Methylethyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; Halogenalkylthio wie vorstehend genannt, insbesondere Difluormethylthio, Trifluormethylthio und Pentafluormethylthio; Halogen wie vorstehend genannt, insbesondere Fluor und Chlor, Cyano oder Nitro;
C₁-C₄-Alkoxy oder -Alkylthio wie unter R¹ genannt, insbesondere Methoxy und Ethoxy, Methylthio und Ethylthio;
C₁-C₄-Halogenalkoxy oder -Halogenalkylthio wie vorstehend genannt, insbesondere Trifluormethoxy, Trichlormethoxy und Pentafluorethoxy, Difluormethylthio, Trifluormethylthio und Pentafluorethylthio;
C₂-C₅-Alkenyloxy wie Vinyloxy, 2-Propenyloxy, 1-Methyl-ethenyloxy, 2-Methyl-3-butenyloxy, insbesondere 2-Propenyloxy und 2-Methyl-3-butenyloxy;
C₂-C₅-Alkinyloxy wie Ethinyloxy, 2-Propinyloxy, 1-Methyl-ethinyloxy, 2-Methyl-3-butinyloxy, insbesondere 2-Propinyloxy und 2-Methyl-3-butinyloxy;
C₁-C₄-Alkylsulfinyl wie Methylsulfinyl, Ethylsulfinyl, n-Propylsulfinyl, i-Propylsulfinyl, n-Butylsulfinyl, tert.-Butylsulfinyl, insbesondere Methylsulfinyl;
C₁-C₄-Alkylsulfonyl wie Methylsulfonyl, Ethylsulfonyl, n-Propylsulfonyl, i-Propylsulfonyl, n-Butylsulfonyl, tert.-Butylsulfonyl, insbesondere Methylsulfonyl;
C₁-C₄-Halogenalkylsulfonyl wie Trifluormethylsulfonyl, Pentafluorethylsulfonyl, Monofluorbutylsulfonyl, insbesondere Trifluormethylsulfonyl;
Phenoxy oder Phenylthio, welches ein- bis dreimal durch C₁-C₄-Alkyl wie unter R¹ genannt, insbesondere Methyl, Ethyl und iso-Propyl; Halogenalkyl wie unter R¹ genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; Halogenalkylthio wie vorstehend genannt, insbesondere Difluormethylthio, Trifluormethylthio und Pentafluormethylthio; Halogen wie vorstehend genannt, insbesondere Fluor und Chlor; Cyano oder Nitro; substituiert sein kann;
ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff wie Tetrahydrofuryl, Tetrahydropyranyl, Furyl, Thienyl, Thiazolyl, Pyrazolyl, Pyrrolyl, Pyridyl, Morpholino, Piperidino, Pyrimidyl, beispielsweise 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furyl, 3-Furyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrazolyl, 4-Pyrazolyl, 5-Pyrazolyl, 2-Pyridyl, 3-Pyridyl und 4-Pyridyl, der ein bis drei der folgenden Substituenten tragen kann: C₁-C₃-Alkyl wie vorstehend genannt, insbesondere Methyl und Ethyl, Halogen wie vorstehend genannt, insbesondere Fluor und Chlor, C₂-C₃-Alkoxy wie unter R¹ genannt, insbesondere Methoxy und Ethoxy; oder C₂-C₄-Alkoxycarbonyl wie Methoxycarbonyl und Ethoxycarbonyl, insbesondere Methoxycarbonyl;
Phenyl, welches eine bis drei der folgenden Gruppen tragen kann: C₁-C₆-Alkyl wie unter R¹ genannt, insbesondere Methyl, Ethyl und iso-Propyl; C₁-C₆-Halogenalkyl wie unter R¹ genannt, insbesondere Trifluormethyl und Chlordifluormethyl; C₁-C₆-Alkoxy wie unter R¹ genannt, insbesondere Methoxy und Ethoxy; C₁-C₆-Halogenalkoxy wie unter R¹ genannt, insbesondere Trifluormethoxy, Trichlormethoxy und Pentafluorethoxy; C₁-C₆-Alkylthio wie unter R¹ genannt, insbesondere Methylthio und Ethylthio; C₁-C₆-Halogenalkylthio wie unter R¹ genannt, insbesondere Difluormethylthio, Trifluormethylthio und Pentafluormethylthio; Halogen wie unter R¹ genannt, insbesondere Fluor und Chlor; Cyano oder Nitro; wobei die Maßgabe gilt, daß einer der drei Substituenten R¹, R³ und R⁴ wie eingangs genannt definiert ist.

R⁴ Wasserstoff bedeutet oder die für R² und R³ genannte Bedeutung haben kann.

Beispiele für bevorzugte Restekombinationen im Pyridinring sind der nachstehenden Tabelle zu entnehmen, wobei R¹ einem Rest aus der Gruppe L1 bis L64 entspricht, insbesondere einen 1-(Cyclopropyl)-ethylrest oder einem Cyclopropylrest.

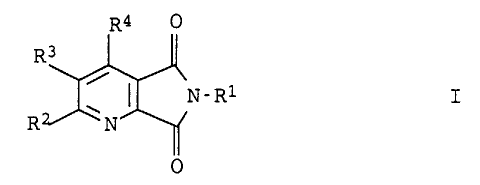

| R² | R³ | R⁴ |
|---|---|---|
| F | F | H |
| F | F | F |
| F | Cl | H |
| F | Cl | Cl |
| F | F | Cl |
| F | Cl | F |
| F | CN | H |
| F | CN | CN |
| F | CN | F |
| F | F | CN |
| F | Cl | CN |
| F | CN | Cl |
| F | CH₃ | H |
| F | CH₃ | CH₃ |
| F | F | CH₃ |
| F | CH₃ | F |
| F | CH₃ | Cl |
| F | Cl | CH₃ |
| F | C₂H₅ | F |
| F | F | C₂H₅ |
| F | CF₃ | H |
| F | Cl | CF₃ |
| F | CF₃ | Cl |
| F | C₂H₅ | H |
| F | CH₂OCH₃ | H |
| F | CH₂OCH₃ | F |
| F | CH₂SCH₃ | H |
| F | CH₂SCH₃ | Cl |
| F | Cl | CH₂SCH₃ |
| F | CH₂-cyclopropyl | H |
| F | 1-(cyclopropyl)ethyl | H |
| F | CH₂CN | H |
| F | CH₃ | CH₂CN |
| F | benzyl | H |
| F | Cl | benzyl |
| F | benzyl | F |
| F | F | benzyl |
| F | 4-chlorbenzyl | H |
| F | 3-CF₃-benzyl | H |
| F | 2,4-Cl₂-benzyl | H |
| F | 3-OCH₃-Phenyl | H |
| F | 3-CN-benzyl | H |
| F | cyclopropyl | H |
| F | cyclopropyl | Cl |
| F | cyclopropyl | F |
| F | CH=CH₂ | H |
| F | F | CH=CH₂ |
| F | CF=CF₂ | H |
| F | CH₃ | CF=CF₂ |
| F | CH₃ | benzyl |
| F | CH=CH-OCH₃ | H |
| F | CH=CH-OCH₃ | Cl |
| F | CH=CH-OCH₃ | F |
| F | CH=CH-phenyl | H |
| F | CH=CH-phenyl | F |
| F | CH=CH-phenyl | Cl |
| F | CH=CH-(3-chlorphenyl) | H |
| F | CH=CH-(3-chlorphenyl) | F |
| F | C≡CH | H |
| F | CH₂-C≡CH | H |
| F | CH₂-C≡CH | F |
| F | CH₃O | H |
| F | CH₃O | F |
| F | CH₃O | Cl |
| F | CH₃O | CH₃ |
| F | Cl | CH₃O |
| F | F | CH₃O |
| F | CH₃ | CH₃O |
| F | CH₃O | CH₃O |
| F | CH₃S | H |
| F | CH₃S | F |
| F | CH₃S | Cl |
| F | CH₃S | CH₃ |
| F | F | CH₃S |
| F | Cl | CH₃S |
| F | CH₃ | CH₃S |
| F | CF₃O | H |
| F | CF₃O | F |
| F | CF₃O | Cl |
| F | F₂CHO | H |
| F | F₂CHO | F |
| F | F₂CHO | Cl |
| F | SCF₃ | H |
| F | SCF₃ | F |
| F | SCF₃ | Cl |
| F | CH₃SO | H |
| F | CH₃SO | F |
| F | CH₃SO | Cl |
| F | CH₃SO₂ | H |
| F | CH₃SO₂ | Cl |
| F | CH₃SO₂ | F |
| F | CF₃SO₂ | H |
| F | CF₃SO₂ | F |
| F | CF₃SO₂ | Cl |
| F | O-phenyl | H |
| F | O-phenyl | F |
| F | O-(4-Cl-phenyl) | H |
| F | O-(3-CF₃-phenyl) | H |
| F | O-(2,4-Cl₂-phenyl) | H |
| F | O-(4-CH₃O-phenyl) | H |
| F | O-(3-CN-phenyl) | H |
| F | S-phenyl | H |
| F | S-(3-Cl-phenyl) | H |
| F | 2-tetrahydrofuranyl | H |
| F | 3-tetrahydrofuranyl | H |
| F | 2-tetrahydrothienyl | H |
| F | 3-tetrahydrothienyl | H |
| F | 2-tetrahydropyranyl | H |
| F | 3-tetrahydropyranyl | H |
| F | 4-tetrahydropyranyl | H |
| F | 2-furyl | H |
| F | 3-furyl | H |
| F | 2-thienyl | H |
| F | 3-thienyl | H |
| F | 3-isoxazolyl | H |
| F | 4-isoxazolyl | H |
| F | 5-isoxazolyl | H |
| F | 3-isothiazolyl | H |
| F | 4-isothiazolyl | H |
| F | 5-isothiazolyl | H |
| F | 2-oxazolyl | H |
| F | 4-oxazolyl | H |
| F | 5-oxazolyl | H |
| F | 2-thiazolyl | H |
| F | 4-thiazolyl | H |
| F | 5-thiazolyl | H |
| F | 2-imidazolyl | H |
| F | 4-imidazolyl | H |
| F | 5-imidazolyl | H |
| F | 2-pyrrolyl | H |
| F | 3-pyrrolyl | H |
| F | 3-pyrazolyl | H |
| F | 4-pyrazolyl | H |
| F | 5-pyrazolyl | H |
| F | 2-pyridyl | H |
| F | 3-pyridyl | H |
| F | 4-pyridyl | H |
| F | phenyl | H |
| F | 4-Cl-phenyl | H |
| F | 2,4-Cl₂-phenyl | H |
| F | 3-CF₃-phenyl | H |
| F | 3-CH₃O-phenyl | H |
| F | Cl | Et |
| F | Cl | CF₃O |
| F | Cl | F₂CHO |
| F | Cl | C₂F₅O |
| F | Cl | CF₃S |
| F | Cl | F₂CHS |
| F | Cl | CH₃SO |
| F | Cl | CH₃SO₂ |
| F | Cl | CF₃SO₂ |
| F | Cl | O-phenyl |
| F | Cl | O-(4-Cl-phenyl) |
| F | Cl | ClCF₂O |
| F | Cl | i-C₃H₇ |
| F | Cl | i-C₄H₉ |
| F | CH₃ | C₂H₅ |
| F | CH₃ | CF₃O |
| F | CH₃ | F₂CHO |
| F | CH₃ | C₂H₅O |
| F | CH₃ | CF₃S |
| F | CH₃ | CH₃SO |
| F | CH₃ | CH₃SO₂ |
| F | CH₃ | CF₃SO₂ |
| F | CH₃ | O-phenyl |
| F | CH₃ | O-(4-Cl-phenyl) |
| F | CH₃ | ClCF₂O |
| F | CH₃ | i-C₃H₇ |
| F | CH₃ | CN |

Bevorzugte Definitionen für R¹ sind:

| Nr. | R¹ | Nr. | R¹ |
|---|---|---|---|
| L1 | OCH₃ | L34 | -C(CH₃)₂-C₂H₅ |
| L2 | OC₂H₅ | L35 | -C(CH₃,C₂H₅) C₂H₅ |
| L3 | O-i-C₃H₇ | L36 | -C(CH₃)₂C₃H₇ |
| L4 | n-C₃H₇ | L37 | -C(CH₃)₂cycloC₆C₁₁ |
| L5 | i-C₃H₇ | L38 | -CH₂-C(CH₃)=CH₂ |
| L6 | n-C₄H₉ | L39 | -CH₂CH=CHCH₃ |
| L7 | i-C₄H₉ | L40 | -CH(CH₃)CH=CHCH₃ |
| L8 | sec-C₄H₉ | L41 | -C(CH₃)₂CH=CHCH₃ |
| L9 | tert.-C₄H₉ | L42 | -CH₂C≡CH |
| L10 | n-C₅H₁₁ | L43 | -CH(CH₃)C≡CH |
| L11 | -CH(CH₃)C₃H₇ | L44 | -C(CH₃)₂C≡CH |
| L12 | -CH(C₂H₅)C₂H₅ | L45 | -C(CH₃,C₂H₅)C≡CH |
| L13 | n-C₆H₁₃ | L46 | -C(C₂H₅)₂C≡CH |
| L14 | -CH(CH₃)C₄H₉ | L47 | -CH₂C≡CCH₃ |
| L15 | -CH(C₂H₅)C₃H₇ | L48 | -CH(CH₃)C≡CCH₃ |
| L16 | OC(CH₃)₃ | L49 | -C(CH₃)₂C≡CCH₃ |
| L17 | cyclo-C₃H₅ | L50 | -CH(CH₃)CH₂SCH₃ |
| L18 | cyclo-C₄H₇ | L51 | -C(CH₃)₂CH₂SCH₃ |
| L19 | cyclo-C₅H₉ | L52 | -CH₂CH₂CH₂SCH₃ |
| L20 | cyclo-C₆H₁₁ | L53 | -CH(CH₃)CH₂Cl |
| L21 | cyclo-C₇H₁₃ | L54 | -C(CH₃)₂CH₂Cl |
| L22 | cyclo-C₈H₁₅ | L55 | -CH(CH₃)CH₂OCH₃ |
| L23 | 1-Methylcyclohexyl | L56 | -C(CH₃)₂CH₂OCH₃ |
| L24 | 1-Ethylcyclohexyl | L57 | -CH₂CH₂CH₂OCH₃ |
| L25 | 3,5-Dimethylcyclohexyl | L58 | -CH₂CH₂CH₂N(CH₃)₂ |
| L26 | 3-Trifluormethylcyclohexyl | L59 | -CH₂CH₂CH₂N(C₂H₅)₂ |
| L27 | 1-(Cyclopropyl)ethyl | L60 | Cyclopropylmethyl |
| L28 | 1-(Cyclopentyl)ethyl | L61 | C(CH₃)₂CH₂F |
| L29 | 1-(Cyclohexyl)ethyl | L62 | H |
| L30 | -CH₂-CH=CH₂ | L63 | CH₃ |
| L31 | -CH(CH₃)CH=CH₂ | L64 | C₂H₅ |
| L32 | -C(CH₃)₂C=CH₂ | L65 | Cyclopropyl-1-methyl |
| L33 | -C(CH₃,C₂H₅)CH=CH₂ | L66 | Cyclopropyl-1-ethyl |

Die Verbindungen I bzw. die sie enthaltenden herbiziden Mittel sowie deren umweltverträgliche Salze z.B. von Alkalimetallen und Erdalkalimetallen können in Kulturen wie Weizen, Reis, Mais, Soja und Baumwolle Unkräuter und Schadgräser sehr gut bekämpfen, ohne die Kulturpflanzen zu schädigen, ein Effekt, der vor allem auch bei niedrigen Aufwandmengen auftritt.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryl- ether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 bis 95 Gew.-%, vorzugsweise zwischen 0,5 bis 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können weiterhin beispielsweise wie folgt formuliert werden:
I. 20 Gew.-Teile der Verbindung Nr. 1.031* werden in einer Mischung gelöst, die aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.
II. 20 Gew.-Teile der Verbindung Nr. 1.031* werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenyl und 10 Gew.-Teilen des Anlagerungsproduktes 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
III. 20 Gew.-Teile des Wirkstoffs Nr. 1.031* werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinus besteht. Durch Eingießen und feines Verteilen der Lösung in 100 000 Gew.-Teilen Wasser erhält man eine wäßrige Dispersion, die 0,02 Gew.-% des Wirkstoffs enthält.
IV. 20 Gew.-Teile des Wirkstoffs Nr. 1.031* werden mit 3 Gew.-Teilen des Natriumsalzes der Diisobutylnaphthalin-α-sulfonsäure, 17 Gew.-Teile des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.
V. 3 Gew.-Teile des Wirkstoffs Nr. 1.031* werden mit 97 Gew.-Teilen feinteiligem Kaolin vermischt. Man erhält auf diese Weise ein Stäubemittel, das 3 Gew.-% des Wirkstoffs enthält.
VI. 20 Gew.-Teile des Wirkstoffs Nr. 1.031* werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

*Die Verbindung ist nicht erfindungsgemäß; sie steht jedoch beispielhaft für die erfindungsgemäßen Verbindungen

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,01 bis 5,0, vorzugsweise 0,05 bis 2,0 kg/ha aktive Substanz (a.S.).

In Anbetracht der Vielseitigkeit der Applikationsmethoden können die erfindungsgemäßen Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium) Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays,

Zur Verbreiterung des Wirkungsspektrums und zur Erzielung synergistischer Effekte können die Pyridin-2,3-dicarbonsäureimide I mit zahlreichen Vertretern anderer herbizider oder wachstumsregulierender Wirkstoffgruppen gemischt und gemeinsam ausgebracht werden. Beispielsweise kommen als herbizide Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazoline, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden oder Wachstumsregulatoren auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellung von Vorprodukten

### a) 6-Chlor-5-methyl-pyridin-2,3-dicarbonsäurediethylester

199,9 g (0,79 mol) 5-Methyl-pyridin-2,3-dicarbonsäurediethylester-N-oxid wurden portionsweise innerhalb 1 Stunde bei 60 bis 70°C in 1 1 Phosphorylchlorid eingeführt und 5 Stunden unter Rückfluß gekocht. Nach dem Abkühlen auf 25°C wurde das Reaktionsgemisch im Vakuum eingeengt und der Rückstand in Methylenchlorid aufgenommen. Nach dem Filtrieren über eine Säure mit Kieselgel und Einengen erhielt man 148,2 g (69 % d. Th.) der Titelverbindung als hellen Sirup, der später erstarrte; Fp. 84 - 86°C.
¹H-NMR (100 MHz), d₆-DMSU: 8,3 δ (s/Ar); 2,43 δ (s/CH₃)

### b) 6-Fluor-5-methyl-pyridin-2,3-dicarbonsäurediethylester

10 g (0,0368 mol) der Verbindung a) wurden in 15 ml Sulfolan vorgelegt, unter Rühren mit 3,2 g (0,055 mol) Kaliumfluorid und 0,3 g 18-Krone-6 versetzt und 2 Stunden bei 160°C gerührt. Nach dem Abkühlen auf 25°C wurde mit 250 ml Wasser versetzt und 3 x mit Methylenchlorid extrahiert. Nach dem Trocknen über Magnesiumsulfat und Einengen im Vakuum erhielt man 8 g der Titelverbindung (85 % d. Th.) als farbloses Öl.
¹H-NMR (100 MHz), CDCl₃: 8,1 δ (d/Ar)

### 4-Chlor-6-fluor-5-methyl-pyridin-2,3 -dicarbonsäure-diethylester und 4,6-Difluor-5-methyl-pyridin-2,3-dicarbonsäure-diethylester

a) Eine Mischung von 15 g (0,049 mol) 4,6-Dichlor-5-methylpyridin-2,3-dicarbonsäurediethylester, 3,3 g (0,056 mol) Kaliumfluorid und 0,25 g 18-Krone-6 in 80 ml Sulfolan wurden 5 Stunden bei 140°C gerüht. Nach dem Abkühlen wurde das Reaktionsgemisch mit 200 ml Methyl-tert.-butylether verdünnt und von den Kaliumsalzen abgesaugt. Das Filtrat wurde 3 mal mit gesättigter Kochsalzlösung extrahiert, über Magnesiumsulfat getrocknet und im Vakuum eingeengt, wobei man 14,2 g eines gelblichen Öls erhielt. Durch Destillation wurden hieraus 3,55 g (27 % d. Th.) der Difluor-Titelverbindung vom Sdp 130 - 138°C/0,5 mbar und 8,39 g (53 % d. Th.) der Chlorfluor-Titelverbindung von Sdp. 148 - 155°C/0,5 mbar isoliert.
b) Unter gleichen Reaktionsbedingungen, jedoch bei Verwendung von 8,5 g (0,148 mol) Kaliumfluorid und 14 Stunden Rühren bei 160°C erhielt man 7,21 g (53 % d. Th.) der Difluor- und 1,17 g (8,3 % d. Th.) der Chlor-fluorverbindung.

### 6-Fluor-pyridin-2,3-dicarbonsäureanhydrid

Eine Mischung von 36,7 g (0,2 mol) 6-Chlor-pyridin-2,3-dicarbonsäureanhydrid, 17,4 g (0,3 mol) Kaliumfluorid und 1 g 18-Krone-6 in 200 ml Propionitril wurde 10 Stunden unter Rückfluß gerührt. Nach dem Abkühlen wurde das Reaktionsgemisch abgesaugt und mit Methylenchlorid gewaschen. Das Filtrat wurde im Vakuum eingeengt und mit einem Gemisch von Methyl-tert.-butylether/Pentan 2:1 verrührt, abgesaugt und getrocknet. Man erhielt 25,3 g (76 % d. Th.) der Titelverbindung vom Fp. 106°C, Zers.

### 5-Chlor-6-methyl-pyridin-2,3-dicarbonsäure-N-tert.-butylimid

### a) 5-Amino-6-methyl-pyridin-2,3-dicarbonsäurediethylester

56,9 g (1,065 mol) Eisenpulver wurden in 200 ml Eisessig vorgelegt, unter Rühren auf 70°C erwärmt und mit einer Lösung von 100 g (0,355 mol) 6-Methyl-5-nitro-pyridin-2,3-dicarbonsäurediethylester (EP 227 932) in 700 ml Eisessig innerhalb 20 Minuten versetzt. Nach 2 Stunden Rühren wurden nochmals 25,2 g (0,45 mol) Eisenpulver in 200 ml Eisessig zugegeben und noch 1 Stunde bei 70°C gerührt. Nach dem Abkühlen auf 25°C, Absaugen des Niederschlages und Einengen des Filtrats im Vakuum wurde der Rückstand in Methylenchlorid aufgenommen, nacheinander mit Wasser, gesättigter Natriumhydrogencarbonat- und Kochsalzlösung extrahiert, über Magnesiumsulfat getrocknet und über eine Nutsche mit Kieselgel abgesaugt. Aus den Fraktionen 3 und 4 isoliert man nach dem Einengen 49 g der Titelverbindung (55 % d. Th.) als gelbes Harz.
¹H-NMR 100 MHz (CDCl₃): 7.13 δ (s/Ar), 4,05 δ (s/NH₂)

### b) 5-Chlor-6-methyl-pyridin-2,3-dicarbonsaurediethylester

5,8 g (0,2 mol) obiger Verbindung a) wurden in 200 ml konz. Salzsaure portionsweise unter Rühren bei 20°C eingetragen und dann auf 0°C abgekühlt. Zu dieser Lösung gab man innerhalb 30 min 31,7 g (0,46 mol) Natriumnitrit, gelost in 60 ml Wasser bei 0 bis 5°C und rührte noch 30 min. Die erhaltene Suspension wurde nun in ein Becherglas mit 29,6 g (0,5 mol) Natriumchlorid, 63,2 g (0,25 mol) Kupfersulfat-5-hydrat und 120 ml Wasser eingeführt und 2 Stunden bei 25°C gerührt. Man extrahierte mit Methylenchlorid, reinigte nacheinander mit gesättigter Natriumhydrogencarbonat- und Kochsalzlösung, trocknete über Magnesiumsulfat und saugte über eine Nutsche mit Kieselgel ab. Nach dem Einengen erhielt man 44,2 g der Titelverbindung (71 % d. Th.) mit n_{D}²³= 1.5150.

### c) 5-Chlor-6-methyl-pyridin-2,3-dicarbonsäureanhydrid

9,5 g (0,038 mol) obiger Verbindung b) wurden in einer Mischung von 3,02 g (0,075 mol) Natriumhydroxid in 20 ml Wasser und 5 ml Ethanol 2 Stunden unter Rückfluß gekocht. Nach dem Abkühlen fallte man mit 500 ml Aceton das Dicarbonsäuresalz aus; 9,1 g (93 % d. Th.) mit Fp. > 240°C.

Hiervon wurden 9 g (0,035 mol) in 100 ml 1,2-Dichlorethan vorgelegt, tropfenweise mit 6,8 g (0,09 mol) Acetylchlorid versetzt und 8 Stunden unter Rückfluß gekocht. Nach dem Abkühlen saugte man ab, wusch mit Methylenchlorid und engte das Filtrat im Vakuum ein. Ausbeute 5,8 g (85 % d. Th.) der Titelverbindung vom Fp.140 - 141°C.

### d) Endstufe

9,9 g (0,05 mol) der Verbindung c) wurden in 150 ml 1,2-Dichlorethan vorgelegt, tropfenweise mit 4,0 g (0,06 mol) tert.-Butylamin versetzt und 3 Stunden bei 70°C gerührt. Nach dem Abkühlen auf 25°C wurden 8,2 g (0,069 mol) Thionylchlorid zugegeben und 14 Stunden gerührt. Man versetzte nacheinander mit 150 ml Methylenchlorid, 150 ml Eiswasser und trennte die Phasen. Nach dem Waschen und Trocknen wie bei b) und dem Einengen erhielt man 10 g 5-Chlor-6-methyl-pyridin-2,3-dicarbonsäure-N-tert.-butylimid (79 % d. Th.) vom Fp. 114 - 115°C (1.031).

### 5-Fluor-pyridin-2,3-dicarbonsäure-N-1-(cyclopropyl)ethylimid

Eine Mischung von 19,5 g (0,0778 mol) 5-Chlor-pyridin-2,3-dicarbonsäure-N-1-(cyclopropyl)ethylimid, 9,1 g (0,156 mol) Kaliumfluorid und 0,4 g 18-Krone-6 in 50 ml Sulfolan wurden 6 Stunden bei 180°C gerührt. Nach dem Abkühlen wurde auf 500 ml Eiswasser gegossen und der ausgefallene Niederschlag abgesaugt und mit Wasser gewaschen. Der Rückstand wurde in Methylenchlorid aufgenommen, über Aktivkohle gerührt und nach dem Trocknen über Magnesiumsulfat über neutrales Aluminiumoxid gesaugt. Nach dem Einengen im Vakuum erhielt man 14,1 g (78 % d. Th.) der Titelverbindung vom Fp. 95 - 98°C.

### Herstellungsbeispiel für die Verbindungen I

### 1. 5,6-Difluor-pyridin-2,3-dicarbonsäure-N-1-(cyclopropyl)ethylimid

Eine Mischung von 7,2 g (0,027 mol) 6-Chlor-5-fluor-pyridin-2,3-dicarbonsäure-N-1-(cyclopropyl)ethylimid, 2,3 g (0,04 mol) Kaliumfluorid und 0,1 g 18-Krone-6 in 50 ml Sulfolan wurden 3 Stunden bei 150°C gerührt. Nach der Aufarbeitung analog Beispiel 2 mit Absaugen über Kieselgel erhielt man 4,8 g (71 % d. Th.) der Titelverbindung vom Fp. 52 - 54°C (Wirkstoffbeispiel 1.046).

Entsprechend dem in Beispiel 1 sowie den Vorprodukten beschriebenen Verfahren wurden die in der Tabelle 1 aufgelisteten Pyridin-2,3-dicarbonsäureimide der Formel I erhalten. In den Tabellen 2 und 3 sind neue Pyridin-2,3-dicarbonsäureanhydride bzw. Pyridin-2,3-dicarbonsäureester aufgeführt.

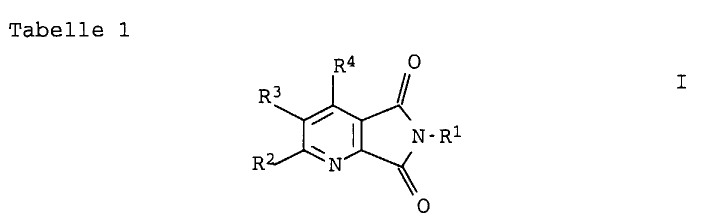

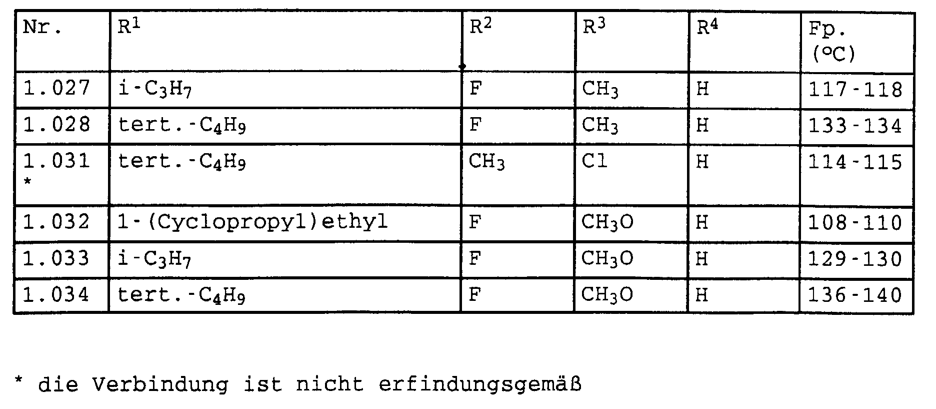

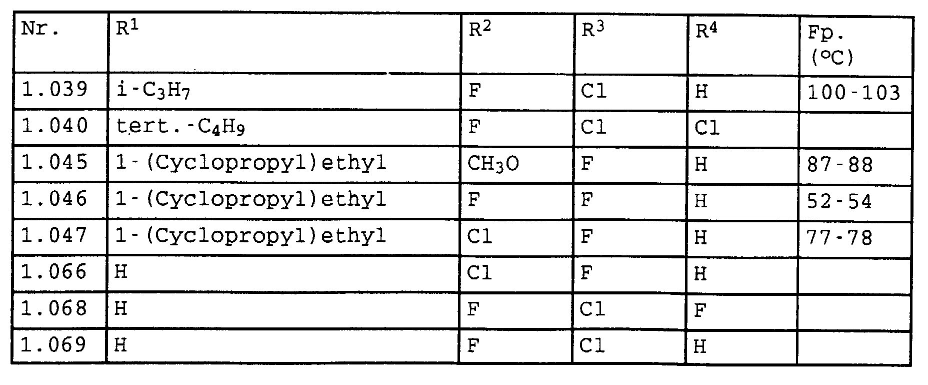

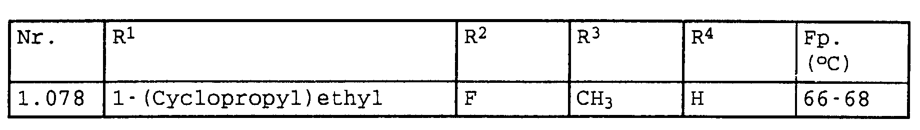

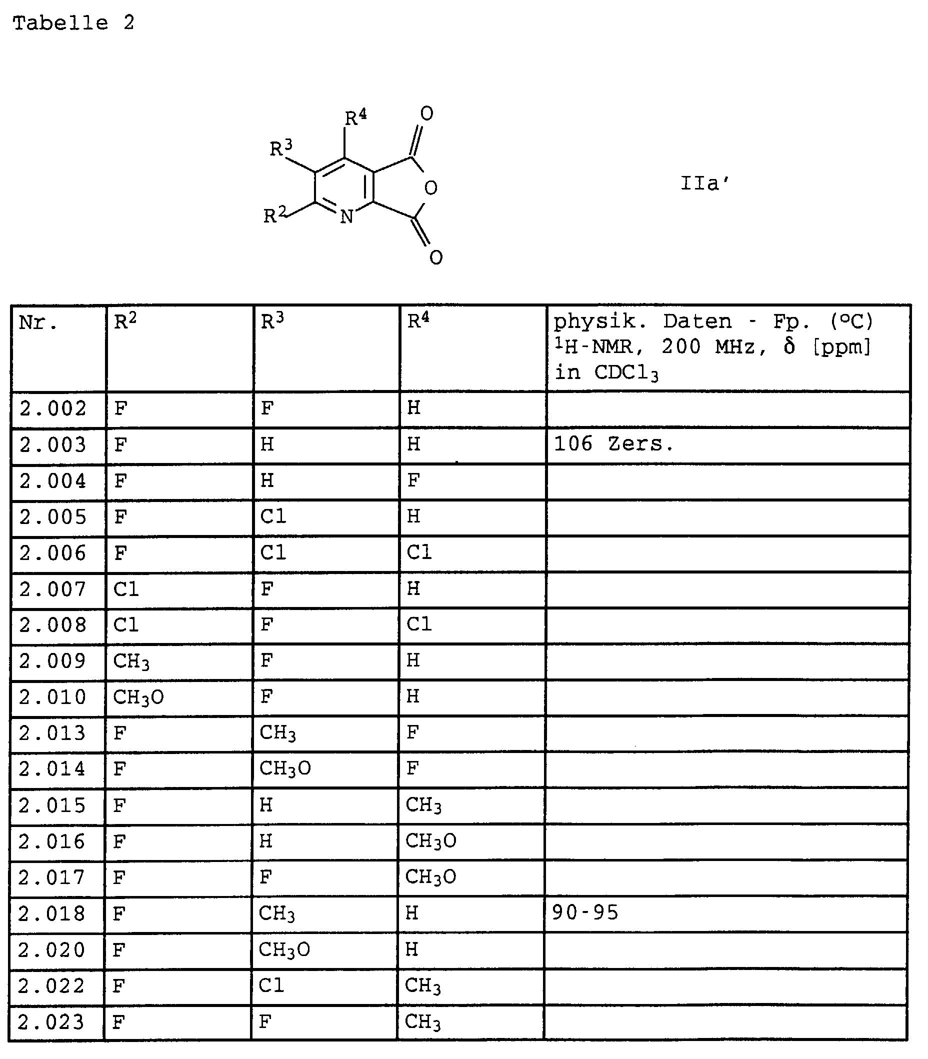

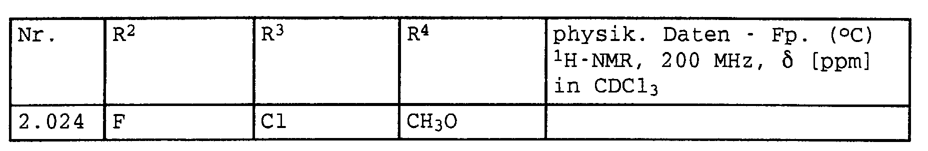

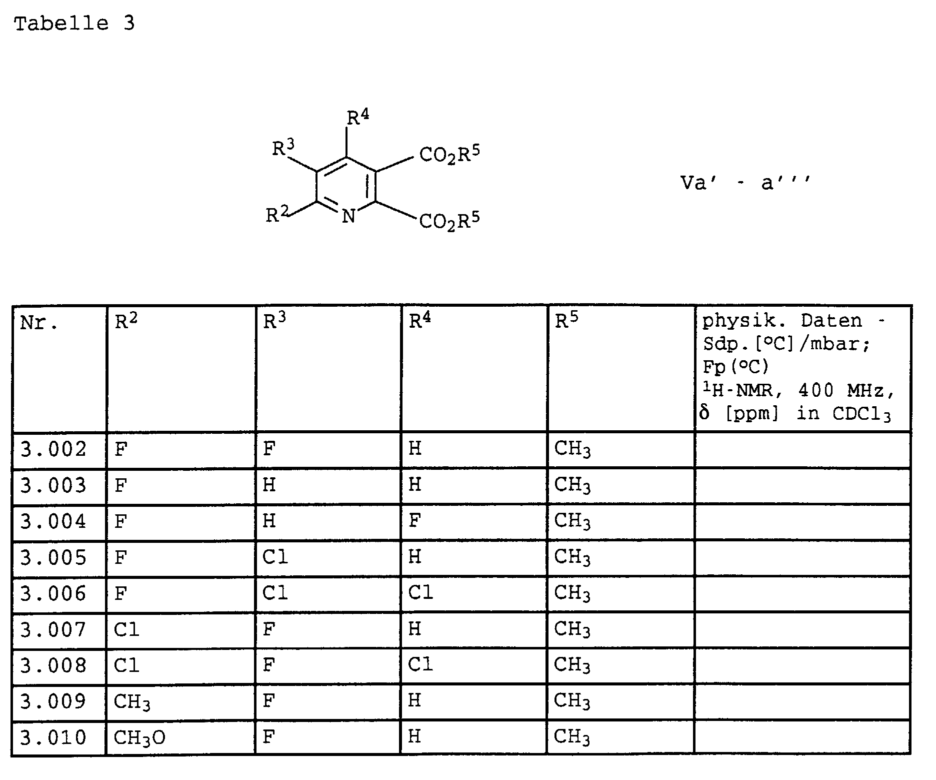

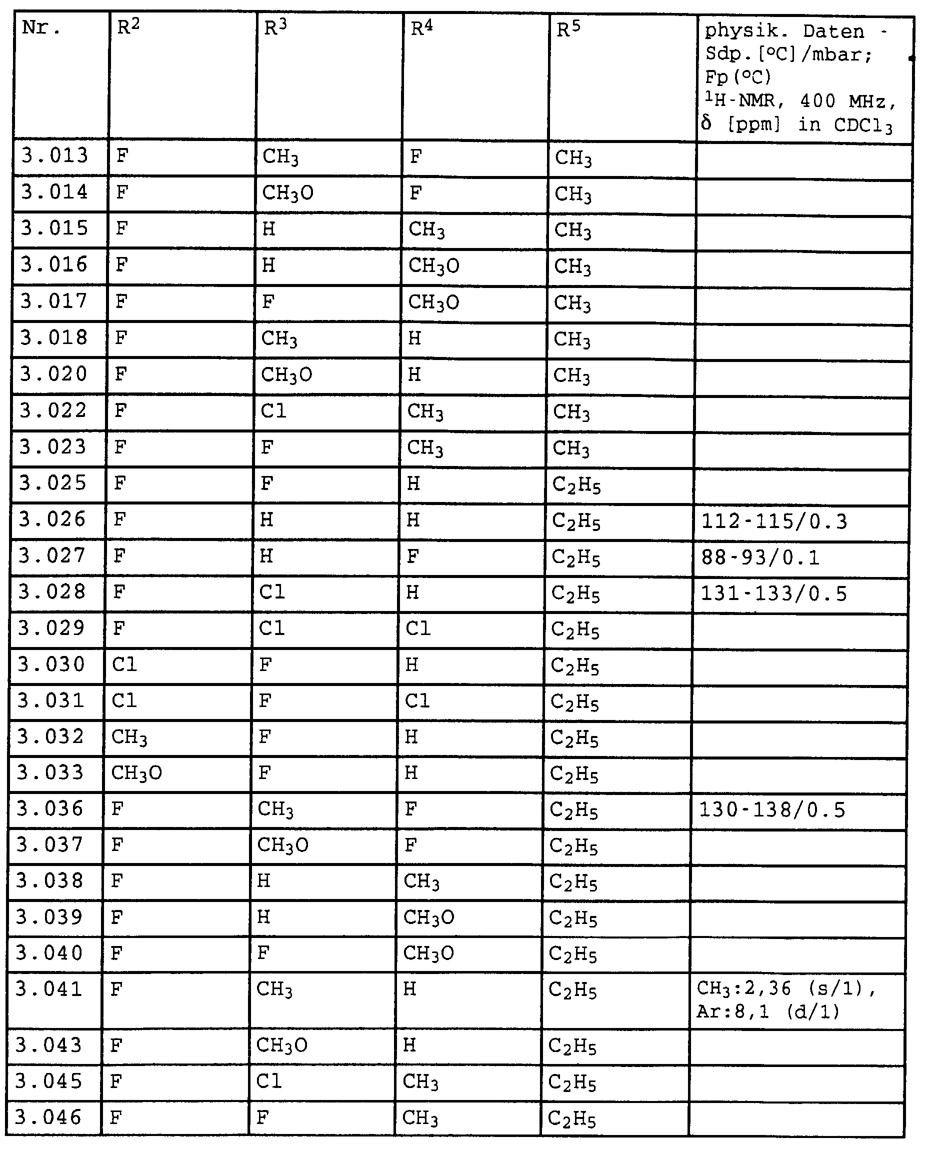

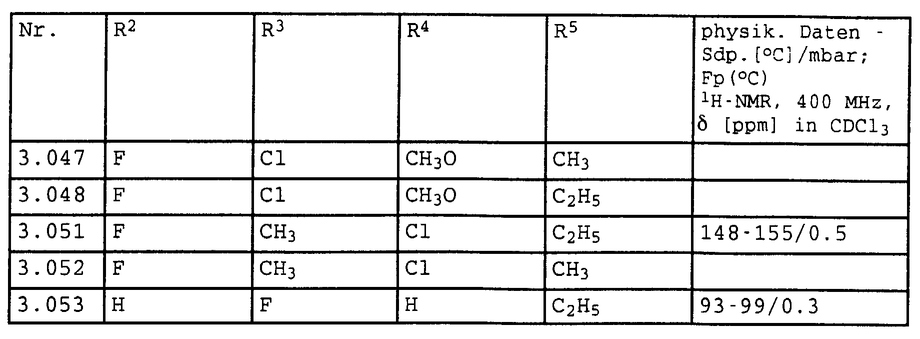

### Anwendungsbeispiele

Die herbizide Wirkung der Pyridin-2,3-dicarbonsäureimide der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren.

Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5 bzw. 0,25 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Die herbizide Wirkung wurde nach einer Skala von 0 bis 100 bewertet. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | | |
|---|---|---|
| Abkürzung | Lateinischer Name | Deutscher Name |
| ABUTH | Abutilon theophrasti | Chinesischer Hanf |
| AMARE | Amaranthus retroflexus | Rauhhaariger Fuchsschwanz |
| CHEAL | Chenopodium album | Weißer Gänsefuß |
| GALAP | Galium aparine | Klettenlabkraut |
| IPOSS | Ipomoea ssp. | Prunkwindenarten |
| STEME | Stellaria media | Vogelmiere |

Die Ergebnisse (s. nachfolgende Tabellen I bis III) zeigen die überlegene herbizide Wirkung der erfindungsgemäßen Verbindungen im Vergleich zu den aus EP-A 422 456 bekannten Vergleichsbeispielen A, B und C.

## Patentansprüche

1. Pyridin-2,3-dicarbonsäureimide der allgemeinen Formel I 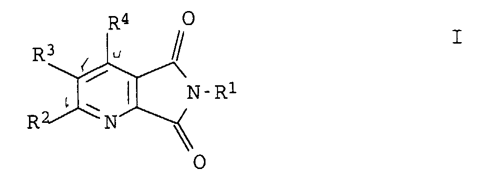 in der
R¹ Wasserstoff;
C₁-C₄-Alkoxy;
C₁-C₆-Alkyl, das eine bis drei der folgenden Gruppen tragen kann: C₁-C₄-Alkoxy, C₁-C₄-Halogenalkoxy, C₁-C₄-Alkylthio, C₁-C₄-Halogenalkylthio, C₁-C₄-Dialkylamino, C₃-C₈-Cycloalkyl, Halogen;
C₃-C₈-Cycloalkyl, das eine bis drei der folgenden Gruppen tragen kann: C₁-C₆-Alkyl, C₁-C₆-Halogenalkyl, C₁-C₄-Alkoxy, C₁-C₄-Halogenalkoxy, Halogen oder Nitro;
C₃-C₆-Alkenyl oder C₃-C₆-Alkinyl, die bis zu dreimal durch Halogen substituiert sein können, bedeutet und
R², und R³ gleich oder verschieden sind und für Halogen, Cyano, C₁-C₆-Alkyl, welches durch ein bis fünf Halogenatome und/oder einen bis zwei der folgenden Reste substituiert sein kann, C₁-C₄-Alkoxy, C₁-C₄-Halogenalkoxy,C₁-C₄-Alkylthio, C₁-C₄-Halogenalkylthio, C₃-C₆-Cycloalkyl oder Cyano;
für Benzyl, das bis zu dreimal durch C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro substituiert sein kann;
für C₃-C₈-Cycloalkyl, das ein- bis dreimal durch C₁-C₄-Alkyl oder Halogen substituiert sein kann;
für C₂-C₆-Alkenyl, welches bis zu dreimal durch Halogen und/oder einmal durch C₁-C₃-Alkoxy oder durch Phenyl, das eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro, substituiert sein kann;
für C₂-C₆-Alkinyl, welches bis zu dreimal durch Halogen oder C₁-C₃-Alkoxy und/oder einmal durch Phenyl, das eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro, substituiert sein kann;
für C₁-C₄-Alkoxy, C₁-C₄-Alkylthio, C₁-C₄-Halogenalkoxy, C₁-C₄-Halogenalkylthio, C₂-C₅-Alkenyloxy, C₂-C₅-Alkinyloxy, C₁-C₄-Alkylsulfinyl, C₁-C₄-Alkylsulfonyl, C₁-C₄-Halogenalkylsulfonyl;
für Phenoxy oder Phenylthio, welche bis zu dreimal durch C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro substituiert sein können;
für einen 5- oder 6-gliedrigen heterocyclischen Rest mit einem oder zwei Heteroatomen, ausgewählt aus der Gruppe Sauerstoff, Schwefel und Stickstoff, der ein bis zwei Substituenten der folgenden Gruppen tragen kann: C₁-C₃-Alkyl, Halogen, C₁-C₃-Alkoxy oder C₂-C₄-Alkoxycarbonyl;
oder
für Phenyl, welches eine bis drei der folgenden Gruppen tragen kann: C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, Halogen, Nitro oder Cyano
stehen, mit der Maßgabe daß mindestens einer der Reste R² und R³ für Fluor steht;
R⁴ Wasserstoff bedeutet oder die für R² und R³ genannte Bedeutung haben kann;
sowie deren umweltverträgliche Salze.

2. Pyridin-2,3-dicarbonsäureimide der Formel I gemäß Anspruch 1, in der die Substituenten folgende Bedeutung haben:
R¹ Wasserstoff C₁-C₆-Alkyl, C₃-C₆-Alkenyl, C₃-C₆-Alkinyl, 1-C₁-C₂-Alkylcyclopropyl-1-(Cyclopropyl)-C₁-C₃-alkyl oder C₃-C₆-Cycloalkyl;
zwei der drei Reste R², R³ und R⁴ unabhängig voneinander C₁-C₄-Alkyl, welches durch ein bis fünf Halogenatome substituiert sein kann, C₁-C₄-Alkoxy, Halogen oder Cyano und mindestens einer der Reste R² und R³ für Fluor stehen.

3. Herbizides Mittel, enthaltend ein Pyridin-2,3-dicarbonsäureimid der Formel I gemäß den Ansprüchen 1 und 2 oder sein Salz sowie hierfür übliche Trägerstoffe.

4. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Pyridin-2,3-dicarbonsäureimids der Formel I gemäß den Ansprüchen 1 und 2 behandelt.

5. Verfahren zur Herstellung von Pyridinderivaten der Formel I gemäß Anspruch 1, dadurch gekennzeichnet, daß man ein Pyridindicarbonsäureanhydrid der Formel II, 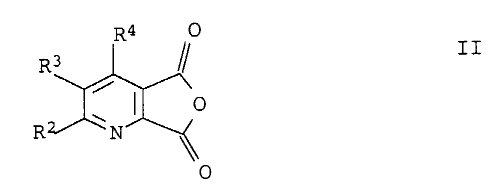 in der die Reste R² bis R⁴ die in Anspruch 1 genannte Bedeutung haben, in an sich bekannter Weise in einem inerten organischen Lösungsmittel mit einem primären Amin III
H₂N-R¹ III
umsetzt und das so erhaltene Pyridindicarbonsäurehalbamid IV 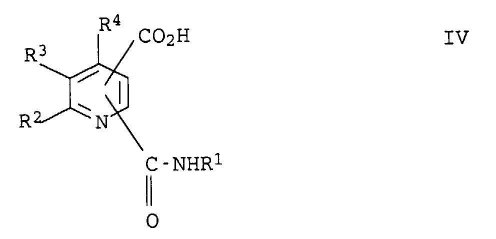 mit wasserabspaltenden Mitteln zu I cyclisiert.

6. Verfahren zur Herstellung von fluorsubstituierten Pyridin-2,3-dicarbonsäureimiden der Formel Ia 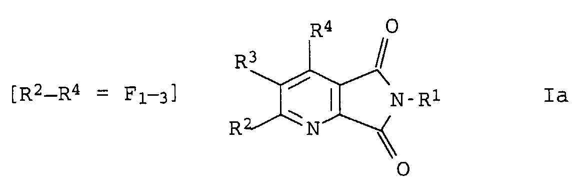 in der R² bis R⁴ die vorgenannte Bedeutung besitzen, wobei mindestens einer der Reste R² oder R³ für Fluor steht, dadurch gekennzeichnet, daß man ein entsprechendes chlorsubstituiertes Pyridin-2,3-dicarbonsäureimid der Formel Ib, 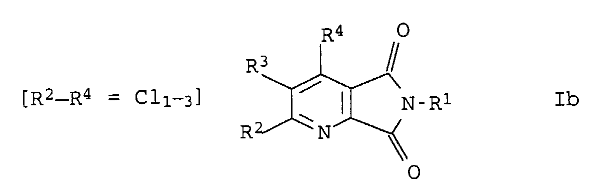 in der R¹ bis R⁴ die vorgenannte Bedeutung besitzen, wobei mindestens einer der Reste R² oder R³ für Chlor steht, mit Kaliumfluorid einem Halogenaustausch unterzieht.

7. Verfahren zur Herstellung von fluorsubstituierten Pyridin-2,3-dicarbonsäureimiden der Formel Ia gemäß Anspruch 7, dadurch gekennzeichnet, daß man das chlorsubstituierte Derivat der Formel Ib in Gegenwart eines aliphatischen Sulfons mit einem Säurechlorid der schwefligen Säure oder Kohlensäure behandelt und dann das Reaktionsgemisch dem Halogenaustausch unterzieht.

8. Verfahren zur Herstellung von fluorsubstituierten Pyridindicarbonsäureanhydriden der Formel IIa 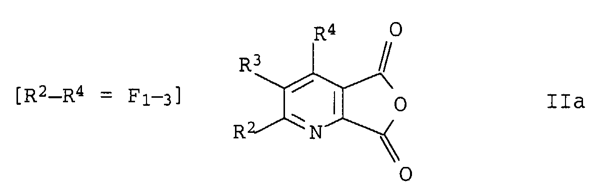 in der R² bis R⁴ die vorgenannte Bedeutung besitzen, wobei mindestens einer der Reste R² oder R³ für Fluor steht, dadurch gekennzeichnet, daß man ein Pyridindicarbonsäureanhydrid der Formel IIb 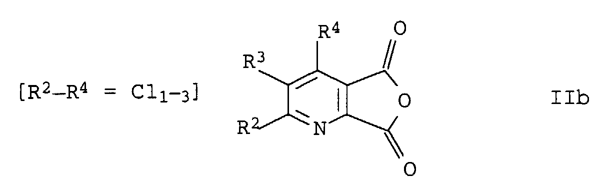 in der R² bis R⁴ die vorgenannte Bedeutung besitzen, wobei mindestens einer der Reste R² oder R³ für Chlor steht, mit Kaliumfluorid einem Halogenaustausch unterzieht.

9. Verfahren zur Herstellung von fluorsubstituierten Pyridindicarbonsäureanhydriden der Formel IIa gemäß Anspruch 9, dadurch gekennzeichnet, daß man das Pyridindicarbonsäureanhydrid der Formel IIb in Gegenwart eines aliphatischen Sulfons mit einem Säurechlorid der schwefeligen Säure oder Kohlensäure behandelt und dann das Reaktionsgemisch dem Halogenaustausch unterzieht.

10. Pyridin-2,3-dicarbonsäureanhydride der Formel IIa' 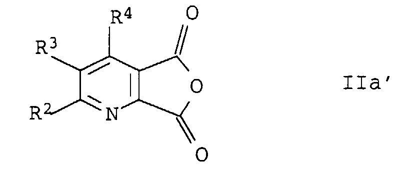 in der einer der Reste R² oder R³ Fluor bedeutet und die übrigen Reste für Wasserstoff, Fluor, Chlor, C₁-C₃-Alkyl, C₁-C₃-Alkoxy, Trifluormethoxy, Chlordifluormethoxy, Methylthio, Trifluormethylthio, Chlordifluormethylthio oder Methylsulfonyl stehen, ausgenommen 4,5,6-Trifluorpyridin-2,3-dicarbonsäureanhydrid.

11. 6-Fluor-pyridin-2,3-dicarbonsäureanhydrid und 5,6-Difluorpyridin-2,3-dicarbonsäureanhydrid.

12. Pyridin-2,3-dicarbonsäureanhydride der Formel IIa' 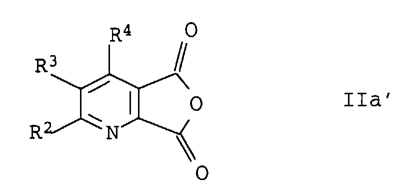 in der R⁴ Wasserstoff bedeutet, R³ für Chlor, C₁-C₃-Alkoxy, Trifluormethyl oder Cyano steht und R² für Fluor steht.

13. Verfahren zur Herstellung von fluorsubstituierten Pyridindicarbonsäureestern der Formel Va 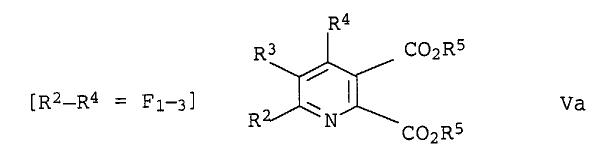 in der R² bis R⁴ die vorgenannte Bedeutung besitzen, wobei mindestens einer der Reste R² oder R³ für Fluor steht, und R⁵ einen ggf. substituierten niedermolekularen Alkyl-, Alkenyl- oder Alkinylrest darstellt, dadurch gekennzeichnet, daß man einen Pyridindicarbonsäureester der Formel Vb, 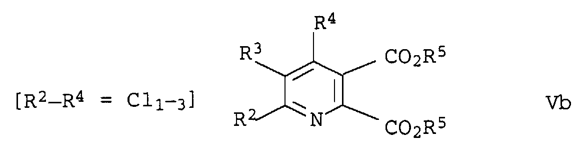 in der R² bis R⁵ die vorgenannte Bedeutung besitzen, wobei mindestens einer der Reste R² oder R³ für Chlor steht, mit Kaliumfluorid einem Halogenaustausch unterzieht.

14. Verfahren zur Herstellung von fluorsubstituierten Pyridindicarbonsäureestern der Formel Va gemäß Anspruch 12, dadurch gekennzeichnet, daß man den Pyridindicarbonsäureester der Formel Vb in Gegenwart eines aliphatischen Sulfons mit einem Säurechlorid der schwefligen Säure oder Kohlensäure behandelt und dann das Reaktionsgemisch dem Halogenaustausch unterzieht.

15. 5,6-Difluor und 4,5,6-Trifluor-pyridin-2,3-dicarbonsäuredialkylester der Formel Va' und Va''' 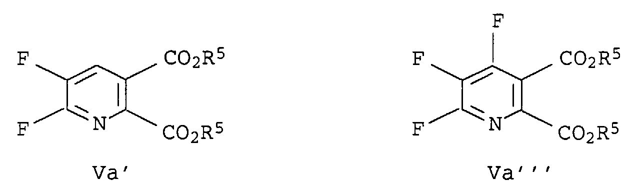 in der R⁵ einen C₁-C₄-Alkyl-, C₃-C₅-Alkenyl- oder C₃-C₅-Alkinylrest, ggf. substituiert durch Halogen, Phenyl oder C₁-C₄-Alkoxy darstellt.

16. Pyridin-2,3-dicarbonsäure-dialkylester der Formel Va'' 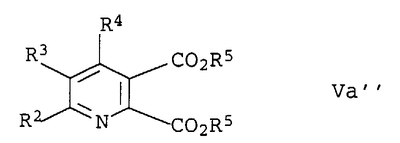 in der einer der Reste R² oder R³ Fluor bedeutet und die übrigen Reste R², R³ oder R⁴ für Wasserstoff, Chlor, C₁-C₃-Alkyl, C₁-C₃-Alkoxy, Trifluormethoxy, Chlordifluormethoxy, Methylthio, Trifluormethylthio, Chlordifluormethylthio oder Methylsulfonyl stehen und R⁵ einen C₂-C₄-Alkyl-, C₃-C₅-Alkenyl- oder C₃-C₅ Alkinylrest, ggf. substituiert durch Halogen, Phenyl oder C₁-C₄-Alkoxy darstellt.

## Claims

1. A pyridine-2,3-dicarboximide of the general formula I 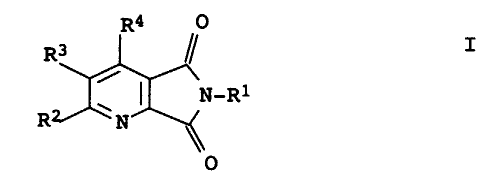 where
R¹ is hydrogen;
C₁-C₄-alkoxy;
C₁-C₆-alkyl which may carry from one to three of the following groups: C₁-C₄-alkoxy, C₁-C₄-haloalkoxy, C₁-C₄-alkylthio, C₁-C₄-haloalkylthio, C₁-C₄-dialkylamino, C₃-C₈-cycloalkyl or halogen;
C₃-C₈-cycloalkyl which may carry from one to three of the following groups: C₁-C₆-alkyl, C₁-C₆-haloalkyl, C₁-C₄-alkoxy, C₁-C₄-haloalkoxy, halogen or nitro;
C₃-C₆-alkenyl or C₃-C₆-alkynyl which may be monosubstituted to trisubstituted by halogen, and
R² and R³ are identical or different and are each halogen, cyano, C₁-C₆-alkyl which may be substituted by from one to five halogen atoms and/or one or two of the following radicals: C₁-C₄-alkoxy, C₁-C₄-haloalkoxy, C₁-C₄-alkylthio, C₁-C₄-haloalkylthio, C₃-C₆-cycloalkyl or cyano;
benzyl which may be monosubstituted to trisubstituted by C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, cyano or nitro;
C₃-C₈-cycloalkyl which may be monosubstituted to trisubstituted by C₁-C₄-alkyl or halogen;
C₂-C₆-alkenyl which may be monosubstituted to trisubstituted by halogen and/or monosubstituted by C₁-C₃-alkoxy or by phenyl which may carry from one to three of the following groups: C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, cyano or nitro;
C₂-C₆-alkynyl which may be monosubstituted to trisubstituted by halogen or C₁-C₃-alkoxy and/or monosubstituted by phenyl which may carry from one to three of the following groups: C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, cyano or nitro;
C₁-C₄-alkoxy, C₁-C₄-alkylthio, C₁-C₄-haloalkoxy, C₁-C₄-haloalkylthio, C₂-C₅-alkenyloxy, C₂-C₅-alkynyloxy, C₁-C₄-alkylsulfinyl, C₁-C₄-alkylsulfonyl, C₁-C₄-haloalkylsulfonyl;
phenoxy or phenylthio, each of which may be monosubstituted to trisubstituted by C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, cyano or nitro;
a 5-membered or 6-membered heterocyclic radical having one or two hetero atoms selected from the group consisting of oxygen, sulfur and nitrogen, which may carry one or two substituents of the following groups: C₁-C₃-alkyl, halogen, C₁-C₃-alkoxy or C₂-C₄-alkoxycarbonyl;
or
phenyl which may carry from one to three of the following groups: C₁-C₆-alkyl, C₁-C₆-haloalkyl, C₁-C₆-alkoxy, C₁-C₆-haloalkoxy, C₁-C₆-alkylthio, C₁-C₆-haloalkylthio, halogen, nitro or cyano,
with the proviso that at least one of the radicals R² and R³ is fluorine;
R⁴ is hydrogen or can have the meanings stated for R² and R³;
and the environmentally compatible salts thereof.

2. A pyridine-2,3-dicarboximide of the formula I as claimed in claim 1,
where
R¹ is hydrogen; C₁-C₆-alkyl, C₃-C₆-alkenyl, C₃-C₆-alkynyl, 1-C₁-C₂-alkylcyclopropyl-1-cyclopropyl-C₁-C₃-alkyl or C₃-C₆-cycloalkyl;
two of the three radicals R², R³ and R⁴, independently of one another, are each C₁-C₄-alkyl which may be substituted by from one to five halogen atoms, or are each C₁-C₄-alkoxy, halogen or cyano, and at least one of the radicals R² and R³ is fluorine.

3. A herbicide containing a pyridine-2,3-dicarboximide of the formula I as claimed in claim 1 or 2 or its salt and conventional carriers.

4. A method for controlling undesirable plant growth, wherein the undesirable plants and/or their habitat are or is treated with a herbicidal amount of a pyridine-2,3-dicarboximide of the formula I as claimed in claim 1 or 2.

5. A process for the preparation of a pyridine derivative of the formula I as claimed in claim 1, wherein a pyridinedicarboxylic anhydride of the formula II 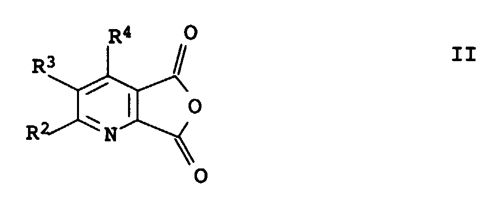 where the radicals R² to R⁴ have the meanings stated in claim 1, is reacted, in a manner known per se in an inert organic solvent, with a primary amine III
H₂N-R¹ III
and the resulting pyridinedicarboxylic acid hemiamide IV 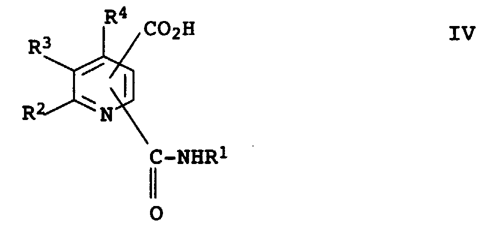 is cyclized with a water-eliminating agent to give I.

6. A process for the preparation of a fluorine-substituted pyridine-2,3-dicarboximide of the formula Ia 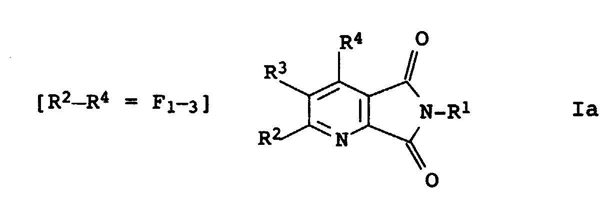 where R² to R⁴ have the abovementioned meanings, at least one of the radicals R² or R³ being fluorine, wherein a corresponding chlorine-substituted pyridine-2,3-dicarboximide of the formula Ib, 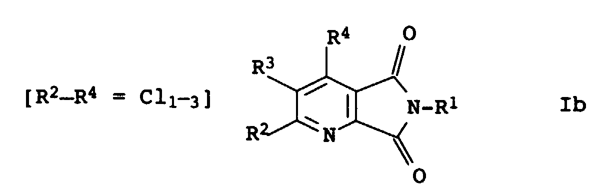 where R¹ to R⁴ have the abovementioned meanings, at least one of the radicals R² or R³ being chlorine, is subjected to halogen exchange with potassium fluoride.

7. A process for the preparation of a fluorine-substituted pyridine-2,3-dicarboximide of the formula Ia as claimed in claim 6, wherein the chlorine-substituted derivative of the formula Ib is treated, in the presence of an aliphatic sulfone, with an acid chloride of sulfurous acid or carbonic acid and the reaction mixture is then subjected to halogen exchange.

8. A process for the preparation of a fluorine-substituted pyridinecarboxylic anhydride of the formula IIa 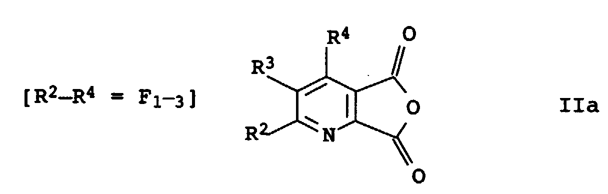 where R² to R⁴ have the abovementioned meanings, at least one of the radicals R² or R³ being fluorine, wherein a pyridinedicarboxylic anhydride of the formula IIb 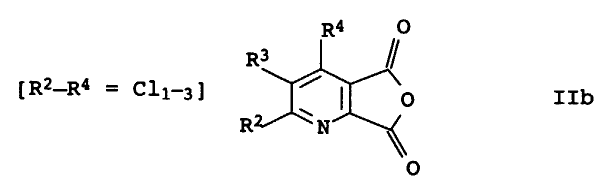 where R² to R⁴ have the abovementioned meanings, at least one of the radicals R² or R³ being chlorine, is subjected to halogen exchange with potassium fluoride.

9. A process for the preparation of a fluorine-substituted pyridinedicarboxylic anhydride of the formula IIa as claimed in claim 8, wherein the pyridinedicarboxylic anhydride of the formula IIb is treated, in the presence of an aliphatic sulfone, with an acid chloride of sulfurous acid or carbonic acid, and the reaction mixture is then subjected to halogen exchange.

10. A pyridine-2,3-dicarboxylic anhydride of the formula IIa' 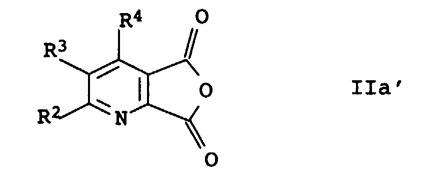 where one of the radicals R² or R³ is fluorine and the other radicals are each hydrogen, fluorine, chlorine, C₁-C₃-alkyl, C₁-C₃-alkoxy, trifluoromethoxy, chlorodifluoromethoxy, methylthio, trifluoromethylthio,
chlorodifluoromethylthio or methylsulfonyl, with the exception of 4,5,6-trifluoropyridine-2,3-dicarboxylic anhydride.

11. 6-Fluoropyridine-2,3-dicarboxylic anhydride and 5,6-difluoropyridine-2,3-dicarboxylic anhydride.

12. A pyridine-2,3-dicarboxylic anhydride of the formula IIa' 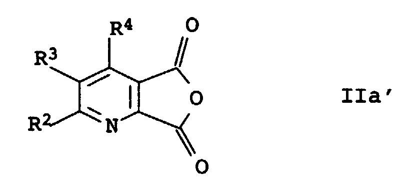 where R⁴ is hydrogen, R³ is chlorine, C₁-C₃-alkoxy, trifluoromethyl or cyano and R² is fluorine.

13. A process for the preparation of a fluorine-substituted pyridinedicarboxylate of the formula Va 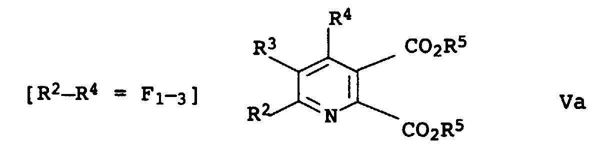 where R² to R⁴ have the abovementioned meanings, at least one of the radicals R² or R³ being fluorine, and R⁵ is an unsubstituted or substituted low molecular weight alkyl, alkenyl or alkynyl radical, wherein a pyridinedicarboxylate of the formula Vb 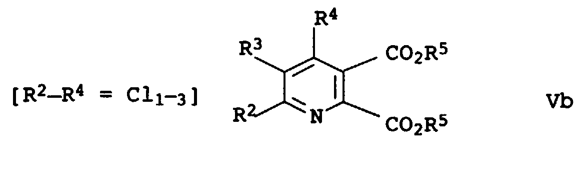 where R² to R⁵ have the abovementioned meanings, at least one of the radicals R² or R³ being chlorine, is subjected to halogen exchange with potassium fluoride.

14. A process for the preparation of a fluorine-substituted pyridinedicarboxylate of the formula Va as claimed in claim 13, wherein the pyridinedicarboxylate of the formula Vb is treated, in the presence of an aliphatic sulfone, with an acid chloride of sulfurous acid or carbonic acid, and the reaction mixture is then subjected to halogen exchange.

15. A dialkyl 5,6-difluoro- or 4,5,6-trifluoropyridine-2,3-dicarboxylate of the formula Va' or Va''' 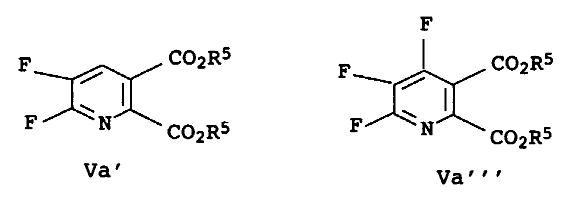 where R⁵ is C₁-C₄-alkyl, C₃-C₅-alkenyl or C₃-C₅-alkynyl, each of which is unsubstituted or substituted by halogen, phenyl or C₁-C₄-alkoxy.

16. A dialkyl pyridine-2,3-dicarboxylate of the formula Va'' 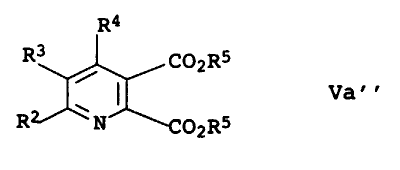 where one of the radicals R² or R³ is fluorine and the remaining radicals R², R³ or R⁴ are each chlorine, C₁-C₃-alkyl, C₁-C₃-alkoxy, trifluoromethoxy, chlorodifluoromethoxy, methylthio, trifluoromethylthio, chlorodifluoromethylthio or methylsulfonyl, R⁴ can also be hydrogen and R⁵ is C₂-C₄-alkyl, C₃-C₅-alkenyl or C₃-C₅-alkynyl, each of which is unsubstituted or substituted by halogen, phenyl or C₁-C₄-alkoxy.

## Revendications

1. Imides d'acides pyridine-2,3-dicarboxyliques de formule générale I 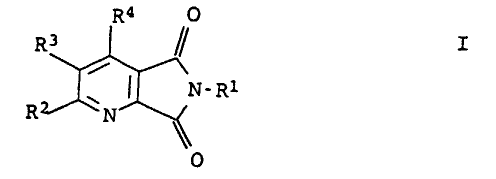 dans laquelle
R¹ représente l'hydrogène ;
un groupe alcoxy en C1-C4 ;
un groupe alkyle en C1-C6 qui peut porter un à trois des substituants suivants : alcoxy en C1-C4, halogénoalcoxy en C1-C4, alkylthio en C1-C4, halogénoalkylthio en C1-C4, di-(alkyle en C1-C4)amino, cycloalkyle en C3-C8, halogéno ;
un groupe cycloalkyle en C3-C8 qui peut porter un à trois des substituants suivants : alkyle en C1-C6, halogénoalkyle en C3-C6, alcoxy en C1-C4, halogénoalcoxy en C1-C4, halogéno ou nitro ;
un groupe alcényle en C3-C6 ou alcynyle en C3-C6 qui peut porter jusqu'à trois substituants halogéno, et
R² et R³, ayant des significations identiques ou différentes, représentent chacun un halogène, un groupe cyano, un groupe alkyle en C1-C6 qui peut porter un à cinq atomes d'halogènes et/ou un à deux substituants suivants : alcoxy en C1-C4, halogénoalcoxy en C1-C4, alkylthio en C1-C4, halogénoalkylthio en C1-C4, cycloalkyle en C3-C6 ou cyano ;
un groupe benzyle qui peut porter jusqu'à trois substituants alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;
un groupe cycloalkyle en C3-C6 qui peut porter un à trois substituants alkyle en C1-C4 ou halogéno ;
un groupe alcényle en C2-C6 qui peut porter jusqu'à trois substituants halogéno et/ou un substituant alcoxy en C1-C3 ou phényle, lequel peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;
un groupe alcynyle en C2-C6 qui peut porter jusqu'à trois substituants halogéno ou alcoxy en C1-C3 et/ou un substituant phényle, lequel peut lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;
un groupe alcoxy en C1-C4, alkylthio en C1-C4, halogénoalcoxy en C1-C4, halogénoalkylthio en C1-C4, alcényloxy en C2-C5, alcynyloxy en C2-C5, alkylsulfinyle en C1-C4, alkylsulfonyle en C1-C4, halogénoalkylsulfonyle en C1-C4 ;
un groupe phénoxy ou phénylthio qui peut porter jusqu'à trois substituants alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;
un radical hétérocyclique à cinq ou six chaînons contenant un ou deux hétéroatomes choisis parmi l'oxygène, le soufre et l'azote qui peut porter un à deux des substituants suivants : alkyle en C1-C3, halogéno, alcoxy en C1-C3 ou alcoxycarbonyle en C2-C4 ;
ou bien
un groupe phényle qui peut porter un à trois des substituants suivants : alkyle en C1-C6, halogénoalkyle en C1-C6, alcoxy en C1-C6, halogénoalcoxy en C1-C6, alkylthio en C1-C6, halogénoalkylthio en C1-C6, halogéno, nitro ou cyano,
sous réserve qu'au moins un des symboles R² et R³ représente le fluor ;
R⁴ représente l'hydrogène ou bien il a l'une des significations indiquées pour R² et R³;
et leurs sels non polluants.

2. Imides d'acides pyridine-2,3-dicarboxyliques de formule I selon la revendication 1 dans laquelle les symboles ont les significations suivantes :
R¹ l'hydrogène,
un groupe alkyle en C1-C6, alcényle en C3-C6, alcynyle en C3-C6, 1-(alkyle en C1-C2)-cyclopropyl-1-(cyclopropyl)-alkyle en C1-C3 ou cycloalkyle en C3-C6 ;
deux des trois symboles R², R³ et R⁴ représentent chacun, indépendamment l'un de l'autre, un groupe alkyle en C1-C4 qui peut porter un à cinq substituants halogéno, un groupe alcoxy en C1-C4, un halogène ou un groupe cyano et au moins un des symboles R² et R³ représente le fluor.

3. Produit herbicide contenant un imide d'acide pyridine-2,3-dicarboxylique de formule I selon les revendications 1 et 2 ou l'un de se sels et des véhicules usuels à cet effet.

4. Procédé pour combattre les croissances de végétaux indésirables, caractérisé par le fait que l'on traite les végétaux indésirables et/ou leur habitat par une quantité herbicide efficace d'un imide d'acide pyridine-2,3-dicarboxylique de formule I selon les revendications 1 et 2.

5. Procédé pour préparer les dérivés de la pyridine répondant à la formule I selon la revendication 1, caractérisé par le fait que l'on fait réagir un anhydride d'acide pyridine-dicarboxylique de formule II 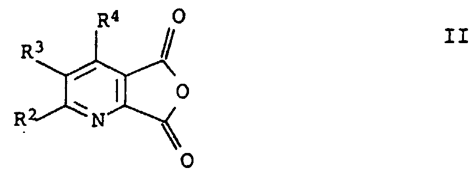 dans laquelle les symboles R² à R⁴ ont les significations indiquées dans la revendication 1, de manière connue en soi, dans un solvant inerte, avec une amine primaire III
H₂N-R¹ III
ce qui donne un hémi-amide d'acide pyridine-dicarboxylique IV 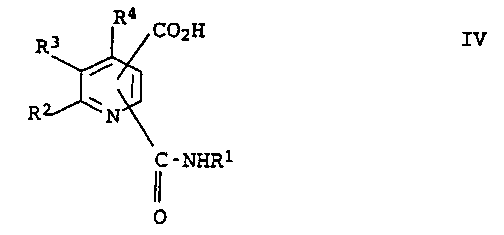 qu'on cyclise en I à l'aide d'agents déshydratants.

6. Procédé pour préparer les imides d'acides pyridine-2,3-dicarboxyliques à substituants fluoro répondant à la formule Ia 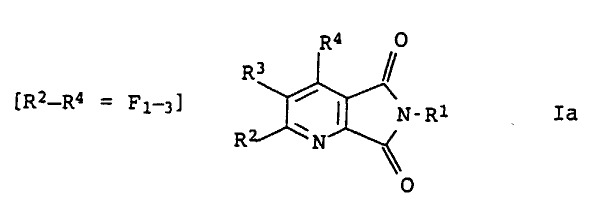 dans laquelle R² à R⁴ ont les significations indiquées ci-dessus, l'un au moins des symboles R² ou R³ représentant le fluor, caractérisé par le fait que l'on soumet un imide d'acide pyridine-2,3-dicarboxylique correspondant à substituant chloro, répondant à la formule Ib 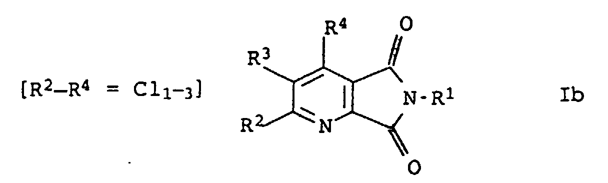 dans laquelle R¹ à R⁴ ont les significations indiquées ci-dessus, l'un au moins des symboles R² ou R³ représentant le chlore, à échange d'halogène à l'aide du fluorure de potassium.

7. Procédé de préparation des imides d'acides pyridine-2,3-dicarboxyliques à substituants fluoro de formule Ia selon la revendication 7, caractérisé par le fait que l'on traite le dérivé à substituants chloro de formule Ib, en présence d'une sulfone aliphatique, par un chlorure d'acide de l'acide sulfureux ou de l'acide carbonique puis on soumet le mélange de réaction à l'échange d'halogène.

8. Procédé de préparation des anhydrides d'acides pyridinedicarboxyliques à substituants fluoro répondant à la formule IIa 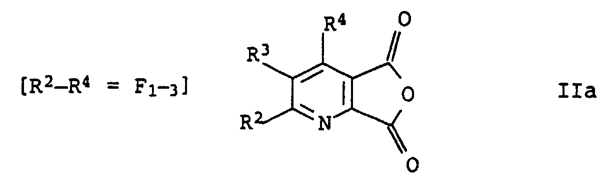 dans laquelle R² à R⁴ ont les significations indiquées ci-dessus, l'un au moins des symboles R² ou R³ représentant le fluor, caractérisé par le fait que l'on soumet un anhydride d'acide pyridine-dicarboxylique de formule IIb 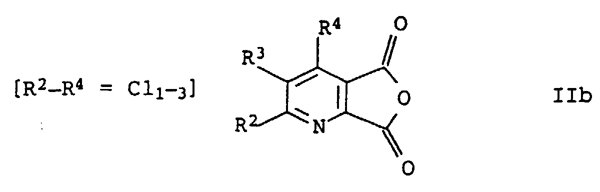 dans laquelle R² à R⁴ ont les significations indiquées ci-dessus, l'un au moins des symboles R² ou R³ représentant le chlore, à échange d'halogène à l'aide du fluorure de potassium.

9. Procédé de préparation des anhydrides d'acides pyridinedicarboxyliques à substituants fluoro de formule IIa selon la revendication 8, caractérisé par le fait que l'on traite l'anhydride d'acide pyridine-dicarboxylique de formule IIb, en présence d'une sulfone aliphatique, par un chlorure d'acide de l'acide sulfureux ou de l'acide carbonique puis on soumet le mélange de réaction à l'échange d'halogène.

10. Anhydrides d'acides pyridine-2,3-dicarboxyliques de formule IIa' 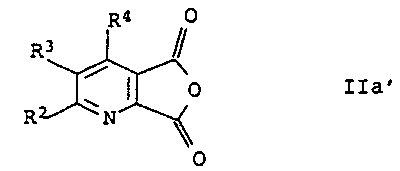 dans laquelle l'un des symboles R² ou R³ représente le fluor et les autres symboles représentent l'hydrogène, le fluor, le chlore, un groupe alkyle en C1-C3, alcoxy en C1-C3, trifluorométhoxy, chlorodifluorométhoxy, méthylthio, trifluorométhylthio, chlorodifluorométhylthio ou méthylsulfonyle, à l'exception de l'anhydride de l'acide 4,5,6-trifluoropyridine-2,3-dicarboxylique.

11. L'anhydride de l'acide 6-fluoropyridine-2,3-dicarboxylique et l'anhydride de l'acide 5,6-difluoropyridine-2,3-dicarboxylique.

12. Anhydrides d'acides pyridine-2,3-dicarboxyliques de formule IIa' 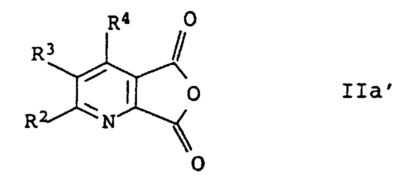 dans laquelle R⁴ représente l'hydrogène, R³ le chlore, un groupe alcoxy en C1-C3, trifluorométhyle ou cyano et R² le fluor.

13. Procédé de préparation des esters d'acides pyridinedicarboxyliques à substituants fluoro répondant à la formule Va 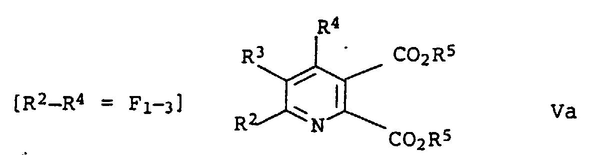 dans laquelle R² à R⁴ ont les significations indiquées ci-dessus, l'un au moins des symboles R² ou R³ représentant le fluor, et R⁵ représente un groupe alkyle, alcényle ou alcynyle inférieur éventuellement substitué, caractérisé par le fait que l'on soumet un ester d'acide pyridine-dicarboxylique de formule Vb 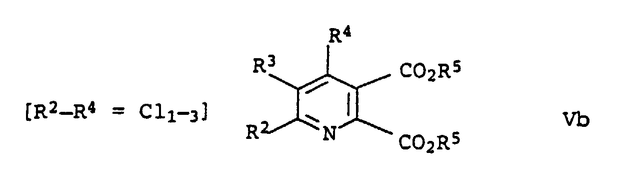 dans laquelle R² à R⁵ ont les significations indiquées ci-dessus, l'un au moins des symboles R² ou R³ représentant le chlore, à échange d'halogène par le fluorure de potassium.

14. Procédé pour préparer les esters d'acides pyridinedicarboxyliques à substituants fluoro de formule Va de la revendication 12, caractérisé par le fait que l'on traite l'ester d'acide pyridine-dicarboxylique de formule Vb, en présence d'une sulfone aliphatique, par un chlorure d'acide de l'acide sulfureux ou de l'acide carbonique puis on soumet le mélange de réaction à échange d'halogène.

15. Les 5,6-difluoro- et 4,5,6-trifluoro-pyridine-2,3-dicarboxylates de dialkyle de formules Va' et Va"' 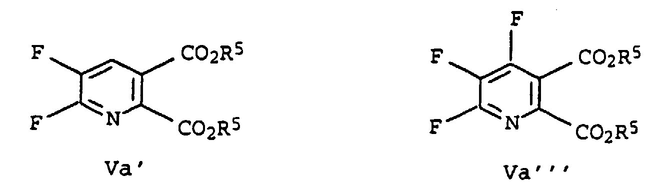 dans lesquelles R⁵ représente un groupe alkyle en C1-C4, alcényle en C3-C5 ou alcynyle en C3-C5 éventuellement substitué par des halogènes, des groupes phényle ou alcoxy en C1-C4.

16. Pyridine-2,3-dicarboxylates de dialkyle de formule Va" 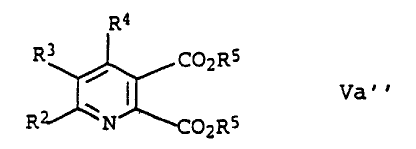 dans laquelle l'un des symboles R² ou R³ représente le fluor et les autres, parmi R², R³ et R⁴, représentent le chlore, des groupes alkyle en C1-C3, alcoxy en C1-C3, trifluorométhoxy, chlorodifluorométhoxy, méthylthio, trifluorométhylthio, chlorodifluorométhylthio ou méthylsulfonyle, R⁴ peut en outre représenter l'hydrogène et R⁵ représente un groupe alkyle en C2-C4, alcényle en C3-C5 ou alcynyle en C3-C5 éventuellement substitué par des halogènes, des groupes phényle ou alcoxy en C1-C4.
